(19) Europäisches Patentamt / European Patent Office / Office européen des brevets

(11) **EP 1 999 250 B1**

(12) **EUROPEAN PATENT SPECIFICATION**

(45) Date of publication and mention of the grant of the patent:
**20.07.2011 Bulletin 2011/29**

(21) Application number: **07702321.6**

(22) Date of filing: **23.01.2007**

(51) Int Cl.:
*C12N 5/0775* (2010.01)  *C12M 3/00* (2006.01)

(86) International application number:
**PCT/CZ2007/000005**

(87) International publication number:
**WO 2007/085210 (02.08.2007 Gazette 2007/31)**

(54) **METHOD OF CULTIVATION OF HUMAN MESENCHYMAL STEM CELLS, PARTICULARLY FOR THE TREATMENT OF NON-HEALING FRACTURES, AND BIOREACTOR FOR CARRYING OUT THIS CULTIVATION METHOD**

VERFAHREN ZUR KULTIVIERUNG MENSCHLICHER MESENCHYMALER STAMMZELLEN, INSBESONDERE ZUR BEHANDLUNG NICHTHEILENDER BRÜCHE, SOWIE BIOREAKTOR ZUR DURCHFÜHRUNG DIESES KULTIVIERUNGSVERFAHRENS

PROCEDE DE CULTURE DE CELLULES SOUCHES MESENCHYMATEUSES HUMAINES, NOTAMMENT POUR LE TRAITEMENT DE FRACTURES A CICATRISATION DIFFICILE ET BIOREACTEUR UTILISE POUR LE PROCEDE DE CULTURE

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.01.2006 CZ 20060049**

(43) Date of publication of application:
**10.12.2008 Bulletin 2008/50**

(73) Proprietors:
- **Univerzita Karlova v Praze**
  **116 36 Praha 1 (CZ)**
- **Ustav Hematologie a Krevni Transfuze**
  **128 20 Praha 2 (CZ)**
- **Ustav Makromolekularni Chemie AV CR, v.v.i.**
  **162 06 Praha 6 (CZ)**

(72) Inventors:
- **PYTLIK, Robert**
  **182 00 Praha 10 (CZ)**
- **HOFMAN, Petr**
  **198 00 Praha 9 (CZ)**
- **TRC, Tomas**
  **147 00 Praha 4 (CZ)**
- **STEHLIK, David**
  **120 00 Praha 2 (CZ)**
- **SOUKUP, Tomas**
  **500 03 Hradec Kralove 3 (CZ)**
- **KOBYLKA, Petr**
  **163 00 Praha 6 (CZ)**

- **KLENER, Pavel**
  **118 00 Praha 1 (CZ)**
- **RYPACEK, Frantisek**
  **196 00 Praha 9 (CZ)**
- **MULINKOVA, Katarina**
  **972 24 Valaska Bela (SK)**

(74) Representative: **Gabrielova, Marta**
**Inventia s.r.o.**
**Na Belidle 3**
**150 00 Praha 5 (CZ)**

(56) References cited:
**WO-A-2005/112542**

- **KIM S J ET AL: "Human adipose stromal cells expanded in human serum promote engraftment of human peripheral blood hematopoietic stem cells in NOD/SCID mice" BIOCHEMICAL AND BIOPHYSICAL RESEARCH COMMUNICATIONS, ACADEMIC PRESS INC. ORLANDO, FL, US, vol. 329, no. 1, 1 April 2005 (2005-04-01), pages 25-31, XP004756992 ISSN: 0006-291X**

- COLTER DAVID C ET AL: "Rapid expansion of recycling stem cells in cultures of plastic-adherent cells from human bone marrow" PROCEEDINGS OF THE NATIONAL ACADEMY OF SCIENCES OF USA, NATIONAL ACADEMY OF SCIENCE, WASHINGTON, DC, US, vol. 97, no. 7, 28 March 2000 (2000-03-28), pages 3213-3218, XP002205331 ISSN: 0027-8424 cited in the application
- KOTOBUKI N ET AL: "Cultured autologous human cells for hard tissue regeneration: Preparation and characterization of mesenchymal stem cells from bone marrow" ARTIFICIAL ORGANS, BLACKWELL SCIENTIFIC PUBLICATIONS, INC., BOSTON, US, vol. 28, no. 1, January 2004 (2004-01), pages 33-39, XP002999366 ISSN: 0160-564X
- KITAMURA S ET AL: "Osteogenic differentiation of human bone marrow-derived mesenchymal cells cultured on alumina ceramics" ARTIFICIAL ORGANS, BLACKWELL SCIENTIFIC PUBLICATIONS, INC., BOSTON, US, vol. 28, no. 1, January 2004 (2004-01), pages 72-82, XP002999370 ISSN: 0160-564X
- KERN SUSANNE ET AL: "Comparative analysis of mesenchymal stem cells from bone marrow, umbilical cord blood, or adipose tissue." STEM CELLS (DAYTON, OHIO) MAY 2006, vol. 24, no. 5, May 2006 (2006-05), pages 1294-1301, XP002432657 ISSN: 1066-5099

**Description**

Technical Field

This invention relates to a method of cultivation of human mesenchymal stem cells in clinical-grade quality, particularly for the treatment of non-healing fractures, as an alternative to existing methods of implantation of autologous or allogeneic bone grafts or autologous, unmanipulated marrow cells.

Background Art

Non-healing fractures are quite common orthopaedic complications, with overall frequency around 3%, but in tibial bones, for example, the frequency of non-healing fractures is 9% and in open fractures combined with the destruction of surrounding soft tissues it may be up to 75% (Csongradi JJ, and Maloney WJ, Ununited lower limb fractures. West J Med 1989; 150: 675-680). Other risk factors predicting for poor healing or non-union of the fracture are smoking, alcohol abuse, obesity, dislocation of bone fragments, osteopenia and the method of surgical treatment used (Chen F et al: Smoking and bony union after ulna-shortening osteotomy. Am. J. Orthop. 2001; 30: 486-489; Foulk DA, and Szabo RM: Diaphyseal humerus fractures: natural history and occurrence of nonunion. Orthopedics. 1995; 18: 333-335; Nilsson LT, et al, Factors predicting healing complications in femoral neck fractures. 138 patients followed for 2 years. Acta Orthop. Scand. 1993; 64: 175-177; Heetveld MJ, et al: Internal fixation for displaced fractures of the femoral neck. Does bone density affect clinical outcome? J Bone Joint Surg Br. 2005; 87: 367-373).

There is no single consensual definition of poor healing or non-union fractures. Some authors consider only the radiological and clinical features (the persistent pain on palpation at the site of fracture, pain on exercise and the absence of bridging bone callus - Sarmiento A, et al: Factors influencing the outcome of closed tibial fractures treated with functional bracing. Clin Orthop. 1995; 315: 8-24). There is not even a consensus among orthopaedic surgeons on the pre-requisite in what time a fracture should heal. In a recently published opinion search among 444 orthopaedic surgeons, the mean time after which the fracture healing was considered as delayed was $3.5 \pm 1.4$ months and the mean time after which the fracture was considered as non-healed was $6.3 \pm 2.1$ months (Bhandari M, et al: A lack of consensus in the asessment of fracture healing among orthopaedic surgeons. J. Orthop. Trauma. 2002; 16: 562-566). Similar time estimations can be found also in orthopaedic textbooks (Bucholz RW: Tibial shaft fractures. In: Orthopaedic Decision Making. 2nd ed. St. Louis MO: Mosby; 1996: 74; Gorczyca JT: Tibial shaft fractures. In: Brinker MR, ed Review of Orthopaedic Trauma. Philadelphia, PA: Saunders; 2001: 127). The time consensus for delayed union or non-union is nevertheless extremely important, because if the fracture is not healed after the expected time, further treatment usually follows.

Among the classical treatment methods of poor-healing fractures belongs adequate immobilization, usually using the external (Green SA, et al: External fixation for the uninfected angulated nonunion of the tibia. Clin Orthop. 1984; 190: 204-211) or internal nailing, autologous or allogeneic bone graft or combination of several of these modalities (Wang JW, and Weng LH: Treatment of distal femoral nonunion with internal fixation, cortical allograft struts, and autogenous bone-grafting. J Bone Joint Surg Am. 2003; 85: 436-440). All of these methods have their disadvantages - external nailing (including the popular Ilizarov's method - Ilizarov GA, et al: Treatment of closed diaphysial fractures of long tubular bones by means of transosseous osteosynthesis. Sov Med. 1983; 9: 21-24) is connected with an increased risk of infection, internal nailing can damage the vascular network at the site of the fracture, the volume of autologous bone graft is limited and the use of allogeneic bone graft leads to a small, but definite risk of infectious or chemical contamination or immune reaction of the host.

In 1989, Connoly first published the results of the treatment of non-healing fractures of long bones with an injection of marrow blood to the site of fracture (Connoly JF, et al: Autologous marrow injection for delayed unions of the tibia: a preliminary report. J Orthop Trauma. 1989; 3: 276-282). Of the first ten patients, nine have been cured and the morbidity of the procedure was significantly lower than would be the expected morbidity of standard surgical procedures using autologous or allogeneic bone grafts. The theoretical rationale of this method is the fact that bone marrow blood contains osteoblastic, osteoclastic and vascular precursors and the local application of these cells can substantially speed up the development of the bridging callus, facilitate the reconstruction of blood vessel supply and contribute to the resorption of necrotic bone. Currently, this method is being paired with the modern methods of the bone marrow blood processing, originally developed for the purposes of bone marrow transplantation in patients with haematological malignancies or immunity defects. Hernigou had treated sixty patients with mononuclear concentrate obtained from 350 ml of bone marrow blood and had assessed the number of osteogenic or mesenchymal progenitor cells, expressed as CFU-F units (i.e., the number of clonogenic mesenchymal cells, capable of colony formation on a plastic adherent surface). While 53 patients, who had had their fractures successfully healed, had received a mean number of 54 962 $\pm$ 17 431 CFU-F to the fracture site, 7 patients, in which the healing had not occurred, had received only 19 324 $\pm$ 6843 CFU-F (p <0,01). Therefore, it is clear that even after the harvest of a quite large volume of the bone marrow blood, the aspirate may not contain enough osteoblastic precursors, needed for the fracture healing (Hernigou P, et al: Percutaneous autologous bone-marrow grafting for nonunions. J. Bone Joint Surg Am. 2005; 87: 1430-1437).

Early osteoblastic precursors are the so called mesenchymal stem cells, or, according to the new nomenclature, marrow stromal cells (MSC). These are rare cells present in the bone marrow with the frequency of one in $10^6$ to one in $10^4$ mononuclear cells, depending on the age of the patient (Werntz JR, et al: Qualitative and quantitative analysis of orthotopic bone regeneration by marrow. J Orthop Res. 1996; 14: 85-93). These cells are able to differentiate into cells of specialized tissues - into the cells of supportive hematopoietic stroma, bone, tendon, cartilage, cardiac muscle, etc.; either after the transfer into the appropriate permissive environment in vivo (Bruder SP, et al: The effect of implants loaded with autologous mesenchymal stem cells on the healint of canine segmental bone defects. J Bone Joint Surg Am. 1998; Jul; 80: 985-996; Jiang Y, et all Pluripotency of mesenchymal stem cells derived from adult marrow. Nature. 2002; 418: 41-49) or after receiving differentiation signals in vitro (Pittenger MF, et al: Multilineage potential of adult human mesenchymal stem cells. Science. 1999; 284: 143-147; Reyes M, et al: Purification and ex vivo expansio of postnatal human marrow mesodermal progenitor cells. Blood 2001; 98: 2615-2625; Makino S, et al: Cardiomyocytes can be generated from marrow stromal cells in vitro. J Clin Invest. 1999; 103; 697-705). These properties of MSC can hopefully lead to new methods of not only the treatment of bone and cartilage defects, but also the treatment of myocardial infarction (Pittenger MF, and Martin BJ. Mesenchymal stem cells and their potential as cardiac therapeutics. Circ Res. 2004; 95: 9-20) or cerebrospinal defects or injuries (Jendelová P, Herynek V, DeCroos J, et al. Imaging the fate of implanted bone marrow stromal cells labeled with superparamagnetic nanoparticles. Magn Reson Med 2003; 50: 767-776).

For the clinical use of the mesenchymal stem cells, the cells have to be quickly expanded from the marrow blood with a method fulfilling the conditions of good manufacturing practice (GMP). The cell preparation according to the good manufacturing practice involves their cultivation in vessels certified for the clinical use, in media certified for the clinical use and in the clean environment certified by the responsible institution (in the Czech Republic, the State Institute for Drug Control - SUKL). In the Czech Republic, it is presently not possible to prepare the MSCs for the clinical use, mainly because of the contemporary practice of mesenchymal stem cells expansion:

1. The method of classical preparation of the mesenchymal stem cells imposes substantive requirements on the purity of the environment. The expansion of mesenchymal stem cells starts with the resuspension of mononuclear cells from bone marrow blood in the culture medium in plastic or glass vessels. The mononuclear cells are then left to adhere to the surface of the vessels for 1-3 days. After this time, the non-adherent cells are removed and fresh medium is added to the adherent cells. The medium is usually changed twice a week (DiGirolamo CM, et al: Propagation and senescence of human marrow stromal cells in culture: a simple colony-forming assay identifies samples with the greatest potential to propagate and differentiate. Br JHaematol. 1999; 107: 275-281; Colter DC, et al: Rapid expansion of recycling stem cells in cultures of plastic-adherent cells from human bone marrow. Proc Natl Acad Sci USA. 2000; 97: 3213-3218). During these manipulations, the cultivation vessels usually have to be opened, which leads to an increased risk of microbial contamination. After 2-3 weeks of cultivation, the bottom of the vessel is covered with 70-90% confluent mesenchymal cells monolayer, whereby from $7.5\text{-}10\times10^6$ bone marrow mononuclear cells it is possible to cultivate $0.4\text{-}1\times10^6$ mesenchymal cells (see Example 3).

2. The classical environment for the cultivation of the MSC is the Dulbecco's Modified Eagle Medium (DMEM) or the Eagle's Minimal Essential Medium in alpha-modification (alpha-MEM), supplemented with 10-20% fetal calf serum (Coelho MJ, Trigo Cabral A, and Fernandes MH: Human bone cell cultures in biocompatibility testing. Part I: osteoblastic differentiation of serially passaged human bone marrow cells cultured in $\alpha$-MEM and in DMEM. Biomaterials. 2000; 21: 1087-1094; Novotová E, Strnadová H, Procházka B, a Pytlik R. In vitro kultivace mezenchymových kmenových buněk u pacientů s lymfoidními malignitami. Transfuse dnes, 2003; 9: 28-34). Neither DMEM, nor alpha-MEM are at this time certified for the clinical use and the use of animal serum is at this time considered to be very problematic because of the possibility of animal disease transmission (e.g., bovine spongiform encephalopathy, BSE) and because of the possibility of severe allergic reactions to the animal protein, especially if the cells should be repeatedly administered to the same patient (Mackensen A, et al: Presence of IgE antibodies to bovine serum albumin in a patient developing anaphylaxis after vaccination with human peptide-pulsed dendritic cells. Cancer Immunol Immunother. 2000; 49: 152-156).

3. Using the classical method, as described above in point 1, it is not possible to get enough MSC for the clinical use during a single expansion. Usually, the cells have to be passaged 1-2 times, which increases the risk of the infectious contamination of the cell culture and also prolongs the time from the bone marrow harvest to the final product for the cellular treatment to 4-6 weeks. Apart from this, it appears that during the passaging, MSC tend to lose their ability to differentiate into specialized tissues (Sugiura F, Kitoh H, Ischiguro N. Osteogenic potential of rat mesenchymal stem cells after several passages. Bioch Biophys Res Comm. 2004; 316: 233-239).

A number of researchers tried to resolve the above-described limitations, but none of them used a complex approach to the problem. As early as in 1995, Gronthos and Simmons have explored the effect of 25 recombinant growth factors on the growth of marrow stromal cells (Gronthos S, and Simmons PJ: The growth factor requirements fo STRO-1-positive human bone marrow stromal precursors under serum-deprived conditions in vitro. Blood. 1995; 85: 929-940). They concluded that the highest numbers of CFU-F can be obtained with the combination of ascorbic acid, dexamethasone, platelet-derived growth factor BB (PDGF-BB) and epidermal growth factor (EGF). Jing-Xiang with co-workers have found that the recombinant human monocyte colony stimulating factor (rh M-CSF) increases the number of CFU-F by 25% and the total number of MSC eight- to tenfold (Jin-Xiang F, et al: Homing efficiency and hematopoietic reconstitution of bone marrow-derived stroma cells expanded by recombinant human macrophage-colony stimulating factor in vitro. Exp Hematol 2004; 32: 1204-1211). Tsutsumi with co-workers have shown that mesenchymal stem cells expanded with fibroblast growth factor 2 (FGF-2) retain better differentiation ability when compared to MSCs expanded without this factor (Tsutsumi S, et al. Retention of multilineage differentiation potential of mesenchymal cells during proliferation in response to FGF. Bioch Bioph Res Comm 2001; 288: 413-419). These works have shown that the use of certain supplements or growth factors, most of which can be produced by recombinant technology, can lead both to a higher yield of mesenchymal stem cells and to the preservation of their ability to differentiate into specialized tissues.

Other authors tried to overcome the need for fetal calf serum in the cultivation medium. Generally, the attempts to cultivate MSC in serum-free conditions were not successful (Hankey DP, et al: Enhacement of human osteoblast proliferation and phenotypic expression when cultured in human serum, Acta Orthop Scand. 2001; 72: 395-403), which is in agreement with our own results (Example 3). Better, but ambiguous results were obtained with human serum or human plasma. In certain studies, the use of human autologous serum or autologous plasma led to better results than the fetal calf serum supplementation (Stute N, et al: Autologous serum for isolation and expansion of human mesenchymal stem cells for clinical use. Exp Hematol. 2004; 32: 1212-1225; Schecroun N, and Delloye Ch: In vitro growth and osteoblastic differentiation of human bone marrow stromal cells supported by autologous plasma. Bone. 2004; 35: 517-524, Kim SJ et al., Biochemical and Biophysical Research Communications, 2005; vol. 329: 25-31), but other studies led to different conclusions (Kuznetsov SA, Mankani MH, and Robey PG. Effect of serum on human bone marrow stromal cells: ex vivo expansion and in vivo bone formation. Transplantation. 2000; 70: 1780-1787). Our own experiments summarized in Example 3 show that these contradictory results might be caused by marked interindividual variability in the cell growth in the presence of pooled human serum without other supplements and that the use of human autologous plasma does not lead to better results than the use of the pooled human serum.

In 2003, Baksh, Davies, and Zandstra have shown that the mesenchymal cells are capable of growth and proliferation also in nonadherent cellular suspension provided that the hematopoietic cells are not removed (Baksch D, Davies JE, and Zandstra PW. Adult human bone marrow-derived mesenchymal progenitor cells are capable of adhesion-independent survival and expansion. Exp Hematol. 2003; 31: 723-732). In 2005, the same authors have proven that the CFU-F and CFU-O expansion (CFU-O are osteoblast colony forming units, a functional test, which similarly to CFU-F shows the number of clonogenic cells capable of forming osteoblast cells colonies) in non-adherent cultures is better when the hematopoietic cells are not removed from the culture (Baksch D, Davies JE, and Zandstra PW. Soluble factor cross-talk between human bone marrow-derived hematopoietic and mesenchymal cells enhances in vitro CFU-F and CFU-O growth and reveals heterogeneity in the mesenchymal progenitor cell compartment. Blood. 2005; 106: 3012-3019). Since it is known that stromal cells and bone cells (osteoblasts) have positive effect on the hematopoietic cell growth (Taichman RS. Blood and bone: two tissues whose fates are intertwined to create the hematopoietic stem-cell niche. Blood 2005; 105: 2631-2639), the assumption that the hematopoietic cells also support the proliferation of the stromal cells seems to be logical. At the same time when the first work mentioned in this paragraph was published, we have begun our own experiments with the co-cultivation of hematopoietic and stromal cells, but in usual adherent cultures. Similarly to Baksh, Davies and Zandstra, we have concluded that when the hematopoietic cells are left in the culture, the stromal cell growth is not compromised, but in fact can be enhanced.

From the evidence recited above, it is clear that certain principles of the cultivation of the mesenchymal stem cells or marrow stromal cells *in vitro* have been discovered. However, none of the works published so far have tried to synthetize the available knowledge with the emphasis on the development of functional and reproducible system of rapid cultivation of the mesenchymal stem cells for the clinical use. The present invention is aimed at the cultivation of sufficient amount of the mesenchymal stem cells in as short time as possible, complying with the good manufacturing practice (GMP) requirements, and without the need for excessive investments, both into the production facilities and into the cultivation process itself. The present invention allows for the production of the mesenchymal stem cells for the therapeutic purposes in existing facilities approved for the processing of blood and hematopoietic cells for the clinical use.

Description of the Invention

The object of the present invention is a method of cultivation of mesenchymal stem cells (marrow stromal cells, MSC), particularly for the treatment of non-healing fractures, from mononuclear marrow blood cells, wherein the cultivation of the cells is carried out in a single step in closed system, in a medium certified for the clinical use, with an addition of 10% human serum and supplements, without animal proteins, without removal of hematopoietic cells and without medium change during the cultivation procedure under the standard conditions for the cultivation of tissue cultures.

It is an aspect of the present invention that dexamethasone, ascorbic acid, human recombinant insulin, human recombinant PDGF-BB, and human recombinant EGF, in combination with human recombinant M-CSF and human recombinant FGF-2, are used as the supplements.

A further aspect of the present invention is that the CellGro™ Hematopoietic Stem Cell medium is used as the medium certified for the clinical use.

It is a further aspect of the present invention that the supplements are added at least once during the cultivation procedure.

Another aspect of the present invention is that the cultivation of the cells is performed for one to three weeks, preferably for 13 to 17 days.

When carrying out the method of the cultivation of mesenchymal stem cells according to the present invention, after the aseptic removal of the mononuclear cells from the marrow blood, said cells are seeded in low density into sterile plastic vessels and cultivated under the standard cell-culture conditions for about one to three weeks in the CellGro™ Hematopoietic Stem Cell Medium, certified for the clinical use, with the addition of 10% human serum and the supplements, without the removal of the non-adherent hematopoietic cells, with at least one further addition of the supplements during the cultivation period, but without the exchange of the cultivation medium and without any other intervention into the closed cultivation system, under the conditions usual for the cultivation of tissue cultures.

The mononuclear cells from marrow blood are obtained in the operation theatre under aseptic conditions using the needle certified for the marrow blood harvest and the syringes certified for the clinical use. The marrow blood clotting is prevented by heparin diluted in normal saline solution. The marrow blood is collected in a certified collection system (e.g, Baxter), the marrow particles are removed using the filters incorporated in the system and erythrocytes are removed from the filtered blood by sedimentation with hydroxyethylstarch. The mononuclear cells are resuspended in a small amount of autologous serum and seeded in the cultivation medium described above.

After two weeks of cultivation, the non-adherent cells are removed together with the medium. The adherent layer is rinsed with sterile buffered saline (phosphate buffered saline, PBS) and detached by enzymatic or non-enzymatic treatment (e.g. with the solution of 0.05% trypsin and 1% EDTA, while preferably TrypLE™ solutions, Pharmingen, which do not contain animal proteins may be used). An adequate amount of fresh cultivation medium is added for the resuspension of the detached cells and eventually for neutralizing the trypsine effect. The cell aggregates are further dissociated by aspiration through a thin injection needle into a sterile syringe and the cell suspension is transferred to a transfusion bag. The cells are centrifuged and rinsed with fresh CellGro™ Hematopoietic Stem Cell Medium. The cell concentration is measured on a hematological analyser and their composition is determined using flow-cytometric measurements. The cells are further diluted with CellGro™ Hematopoetic Stem Cell Medium to get the required concentration and ready for use.

The cultivation of the cells according to the present invention can be carried out in a bioreactor containing plastic single-use vessels, which are subsequently placed in a $CO_2$ incubator with maintained inner atmosphere containing 5% $CO_2$ and at the temperature 37 ˚C.

The present invention brings the following unique and original solutions:

a) for the first time, it proves the possibility of the cultivation of mesenchymal cells in the cell culture medium certified for the clinical use and shows that this medium in combination with human serum, human recombinant growth factors and other supplements leads to higher yields of mesenchymal stem cells than the usual research-grade culture medium alfa-MEM;

b) for the first time, it shows that a sufficient amount of mesenchymal cells for the clinical use (including a sufficient amount of CFU-F necessary for bone healing) can be obtained from the marrow blood during the single-step, approximately two weeks long cultivation procedure, in a closed system, and without the removal of the hematopoietic and the non-adherent cells, as well as without the need for the exchange of the cultivation medium;

c) logically connects all the steps necessary for the clinical-grade mesenchymal stem cell cultivation, from the aseptic marrow blood aspiration to the final product;

d) provides, after certain changes of culturing conditions, for the preparation of the mesenchymal stem cells also for other than orthopaedic or traumatologic purposes.

The present invention thus brings a novel method of the mesenchymal stem cells manipulation, whereby these cells are from the marrow blood aspiration to the final product release treated in the closed system and processed by routine methods used for the preparation of conventional transfusion medicine products.

Brief description of the drawings

In Figure 1, the growth of mesenchymal stem cells in alpha-MEM with fetal calf serum is shown (1a - after one week of cultivation, 1b - after two weeks of cultivation).

In Figure 2, the growth of mesenchymal cells in CellGro™ Hematopoietic Stem Cell Medium with human serum and the supplements is shown (2a - after one week of cultivation, 2b - after two weeks of cultivation)

Figure 3 shows the calculation of the percentage of the mesenchymal stem cells (CD45$^{neg}$CD235$^{neg}$) in the total number of the harvested adherent cells. The mesenchymal stem cells are shown as green, the leukocytes (CD45$^+$) as blue and the erythroid precursors (CD235a$^+$) red. FSC = forward scatter, SSC = side scatter. CD90 is one of the surface markers of the mesenchymal cells, but is not necessarily present on all mesenchymal cells, and also it is not specific for these cells.

Figure 4 shows the yields of the mesenchymal cells grown in different media with different numbers of supplements. When the yields of the cells grown in CellGro™ Hematopoietic Stem Cell Medium with human serum and the seven supplements are compared to all other media formulations (with the exception of CellGro™ Hematopoietic Stem Cell Medium with autologous plasma and the seven supplements), the differences are statistically significant.

Figure 5 shows the numbers of CFU-F obtained by the primary expansion of the mesenchymal stem cells in alpha-MEM with fetal calf serum or in CellGro™ Hematopoietic Stem Cell Medium with human serum and the supplements. Because of the small number of experiments, the differences are not statistically significant (with the exception of the columns marked by asterisks). Paired t-test was used for the testing of statistical significance.

Figure 6 shows the alkaline phosphatase production by the cells grown in two-dimensional cultures in osteogenic induction medium. 6a - cells obtained by the primary expansion in alpha-MEM with fetal calf serum, 6b - cells obtained by the primary expansion in CellGro™ Hematopoietic Stem Cell Medium with human serum and the supplements (alkaline phosphatase reaction stained blue, with neutral red background staining).

Figure 7 is the native photography of the growth of the cells on polylactide scaffolds. 7a - day 6 of the culture, 7b - day 13 of the culture. The extracellular matrix is marked by the arrow.

Figure 8 shows the cell growth on the three-dimensional polylactide scaffolds and the production of matrix (I). 8a - half-thin plastic-resin embedded slide, panoptical staining (cells shown by the long arrow, matrix by the short arrow, the black lines are artefacts - air bubbles). 8b - paraffin-embedded slide, staining for the extracellular matrix (osteoid, blue).

Figure 9 shows the cell growth on the three-dimensional polylactide scaffolds and the production of matrix (II). 9a - collagen staining (van Gieson, red), 9b - immunohistochemical staining for osteonectin (brown, with background green trichrome staining).

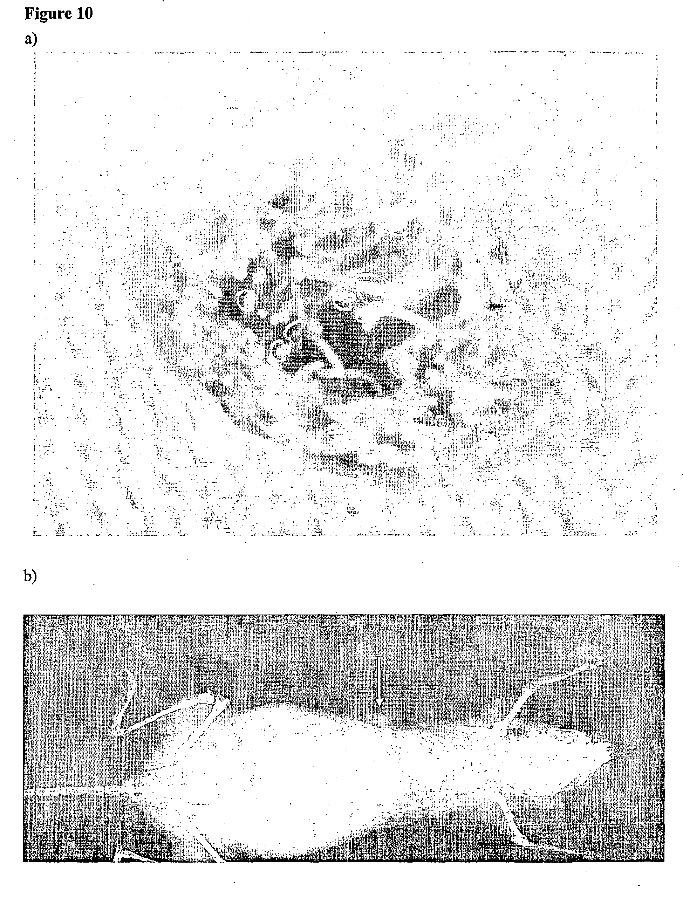

Figure 10 shows the bone formation by the cells on the three-dimensional scaffolds (III). 10a - stereomicroscopical picture of the three-dimensional polylactide scaffold (von Kossa staining, brown). 10b - x-ray of an immunodeficient NOD/LtSz-*Rag1$^{null}$* mouse with two subcutaneously implanted scaffolds. The scaffold on the upper side of the picture was seeded with the cells obtained by the primary expansion in CellGro™ Hematopoietic Stem Cell Medium with human serum and the supplements and is visible as the small nontranslucent rectangle (white arrow). The scaffold in the left flank (lower side of the picture) is control with no seeded cells and is not visible.

Figure 11 shows the elemental analysis of the extracellular matrix. 11a - electronmicroscopic picture of the site of analysis, 11b - chart of the elemental analysis with the peaks of carbon (C), oxygen (O), calcium (Ca) and phosphorus (P) shown.

Figure 12 shows the bone formation on the three-dimensional polylactide scaffolds seeded with the cells obtained by the primary expansion in CellGro™ Hematopoietic Stem Cell Medium with human serum and the supplements and grown subcutaneously in the immunodeficient NOD/LtSz-*Rag1$^{null}$* mice. Among the grey-brown polylactide fibres, the calcified matrix is stained red, the noncalcified matrix (osteoid) is blue, 12a - small magnification, 12b-large magnification.

Figure 13 shows the perspective view of the bioreactor according to example 10

Figure 14 shows the front view of the bioreactor of example 10.

Figure 15 shows the side view of the bioreactor of example 10.

Figure 16 shows the perspective view of the carrier of the bioreactor of example 10, in which two cultivation vessels are inserted for illustration purposes.

Figure 17 shows the perspective view of the cultivation vessel of example 10.

Figure 18 shows the front view of the cultivation vessel, figure 19 shows the ground view and figure 20 the side view of the cultivation vessel of example 10.

<u>Examples</u>

1. Research subj ects

Two groups of research subjects were used. The first group consisted of patients with peripheral artery occlusive disease, in which the marrow blood was harvested and processed to obtain the mononuclear fraction under aseptic conditions for therapeutic purposes. Samples of the mononuclear fraction were used for the mesenchymal stem cells cultivation in the media for the growth of mesenchymal stem cells. The second group consisted of patients with suspected or proven haematological disease who underwent the bone marrow aspiration for diagnostic or staging purposes. The mesenchymal cells are generally considered to be normal in these patients and therefore we considered them suitable for the purposes of our research *(*Soenen-Cornu V, et al: Mesenchymal cells generated from patients with myelodysplastic syndromes are devoid of chromosomal clonal markers and support short- and long-term hematopoiesis in vitro. Oncogene. 2005; 24: 2441-2448*).* Studied subjects were intentionally selected on the basis of age, which should be similar to the potential target group of orthopaedic patients with poorly healing fractures. Therefore, on the contrary to similar experiments, which have been performed mostly with younger subjects, the majority of our samples was obtained from the subjects 50 to 80 years old. All procedures were performed in the General University Hospital, Prague, Czech Republic and were approved by the local review board. All research subjects have given their written informed consent to all routine and research procedures. The demographic characteristics of the research subjects are given in Example 1.

2. Aseptic marrow blood mononuclear cell harvest

In the patients with peripheral artery occlusive disease, the marrow blood harvests were performed for therapeutic use. The mononuclear cells (i.e. the marrow cells depleted of red cells and mature myeloid cells; this population contains immature hematopoietic cells, vascular precursor cells, mesenchymal cells and osteoblast precursors) were intended for an intra-arterial infusion to the limb suffering from peripheral artery occlusive disease, where these cells support the collateral blood vessel development. In the patients under analgosedation or in epidural anesthesia, under usual aseptic conditions, approximately 350 ml of bone marrow blood in 3-4 ml portions was harvested from one or more skin punctures from both posterior iliac crests. The marrow blood clotting was prevented with the normal saline-heparin solution. The marrow blood was collected in the certified Bone Marrow Collection Kit with Pre-Filter and Inline Filters (Baxter R4R2107) which have indwelling filtres for removal of large marrow particles. After the filtration of the blood from the collection bag to the transport and processing bag, the blood was transferred into the facility certified for its processing. The appropriate amount of Gelofusin (B. Braun, Melsungen, AG) was added directly to the processing bag and repeated red cell sedimentation was performed according to the standard operational procedure for approximately 2 hours. The supernatant plasma, containing mainly nuclear cells and only a minimum amount of erythrocytes, was segregated by plasmaextractor and centrifuged again. Clear plasma was then transferred back to the sedimentation bag and the process was repeated. Then the resulting mononuclear cell fraction was resuspended in a small amount of autologous plasma, concentrated as appropriate and prepared for the intraarterial infusion or for further cell expansion in the bioreactor. The procedure allows for more than 90% recovery of marrow mononuclear cells with less than 3% erythrocyte contamination. The results of the marrow blood harvest and the mononuclear fraction extraction are shown in Example 2.

3. Preparation of the reagents and the mesenchymal stem cells cultivation

Research-grade cultivation media and other reagents (alpha-MEM, EDTA-trypsin solution, glutamine, antibiotic solutions and fetal calf serum) were purchased from Gibco (Invitrogen division, Paisley, Scotland). Clinical grade Cell-Gro™ Medium for Hematopoietic Stem Cells was kindly donated by CellGenix (Freiburg, Federal Republic of Germany). Human plasma AB Rh negative was purchased from the blood bank of the Institute of Hematology and Blood Transfusion (Prague, Czech Republic).

Further, the following supplements were used: water-soluble dexamethasone was purchased from Sigma-Aldrich (Steinheim, Federal Republic of Germany), human recombinant insulin for therapeutic use from Eli Lilli (Prague, Czech Republic), ascorbic acid (vitamin C) for clinical use from Biotica (Prague, Czech Republic), human recombinant epidermal growth factor (EGF) and human recombinant platelet-derived growth factor BB (PDGF-BB) from BD Biosciences (Bedford, Massachussetts, USA), recombinant human fibroblast growth factor 2 (FGF-2, research-grade) from Invitrogen (Eugene, Oregon, USA) and recombinant human macrophage colony-stimulating factor (CSF-1, M-CSF, research-grade) from R&D (Minneapolis, Minnesota, USA). All research-grade growth factors were lyophilised and carrier-free (e.g., without bovine albumin) and were reconstituted by tissue grade distilled water with clinical grade human albumin in normal saline (Baxter AG, Vienna, Austria). The choice of the companies delivering the research grade media, other reagents and supplements, and the choice of specific products were directed solely by their availability. The human recombinant proteins did not contain any animal albumin impurities, and, where possible, clinical grade preparations were used.

The human serum was obtained by plasma recalcification according to Dyr. Aliquots of human AB Rh negative plasma from five different donors were pooled to adjust for the interindividual donor variability and, if possible, to receive a homogenous batch for the whole series of experiments. The human plasma was mixed with 0.1 M $CaCl_2$ in 9:1 ratio and incubated for 180 minutes at room temperature. After removing the fibrin clot, the product was incubated for further 48 hours at 4°C. The remaining fibrin was removed by the filtration through metal strainer, the serum was sterilized by the filtration through a 0.22 $\mu$m filter and the aliquots were stored at the temperature of - 80 °C. The described procedure was chosen with regard to the ease of carrying out the procedure, and because it is commonly used. Certainly, other similar procedures can be exercised for the preparation of the human serum. By using this procedure, we have intentionally avoided the use of animal thrombin for defibrination.

Mesenchymal stem cells were cultivated in different combinations of media, sera and supplements. Mononuclear marrow cells were seeded in the concentration ranging from $2.5x10^6$ to $10x10^6$ cells in 10 ml of the complete culture medium in 75 $cm^2$ plastic cultivation flask (i.e., in the densities of $33x10^3$ to $133x10^3$ cells/$cm^2$). When the human serum and the supplements were added to the basal medium, no differences in the mesenchymal stem cells yield were found with the seeding concentrations ranging from $2.5x10^6$ to $7.5x10^6$ mononuclear cells in 10 ml of complete medium in 75 $cm^2$ cultivation flask. In the media supplemented with fetal calf serum, no differences in the mesenchymal cells yield were observed with the seeding concentrations ranging from $7.5x10^6$ to $10x10^6$ mononuclear cells in 10 ml of complete medium in 75 $cm^2$ cultivation flask. Therefore, in the subsequent experiments, $2.5x10^6$ mononuclear marrow cells were seeded to the media with human serum and the supplements, but $10x10^6$ mononuclear marrow cells were seeded in the media with fetal calf serum without the supplements.

The following six culture media were used:

a. alpha-MEM + 10% fetal calf serum + 2% glutamine +1% penicillin, streptomycin and amphotericin B solution.
b. alpha-MEM + 10% human serum + 2% glutamine +1% penicillin, streptomycin and amphotericin B solution.
c. alpha-MEM + 10% human serum + 2% glutamine +1% penicillin, streptomycin and amphotericin B solution and other supplements.
d. CellGro™ for Hematopoietic Stem Cells +1% penicillin, streptomycin and amphotericin B solution and other supplements.
e. CellGro™ for Hematopoietic Stem Cells + 10% human serum + 1% penicillin, streptomycin and amphotericin B solution and other supplements.
f. CellGro™ for Hematopoietic Stem Cells + 10% autologous plasma + 1% penicillin, streptomycin and amphotericin B solution and other supplements.

The following "other supplements" were used:

Five basic supplements according to Gronthos and Simmons (see above):

10 ng/ml EGF
100 ng/ml PDGF-BB
0.25 U/ml human insulin

0.01 $\mu$M dexamethasone
100 $\mu$M vitamin C.

Gronthos and Simmons have tested a wide panel of growth factors and hormones in different combinations and concentrations. Thus, we have taken over the formula, which proved to be the best one, and we did not validate different ratios of the supplements. This formula was published and is available.

Two other supplements were added sequentially. In the first step, 25 ng/ml M-CSF was added. Jin-Xiang in his work recited above used 50 ng/ml, but he did not use the other supplements. The amount of M-CSF was selected empirically. In the second step, 1 ng/ml FGF-2 was added according to the Tsutsumi work. This work was published and the amount of FGF-2 is not the object of the present invention.

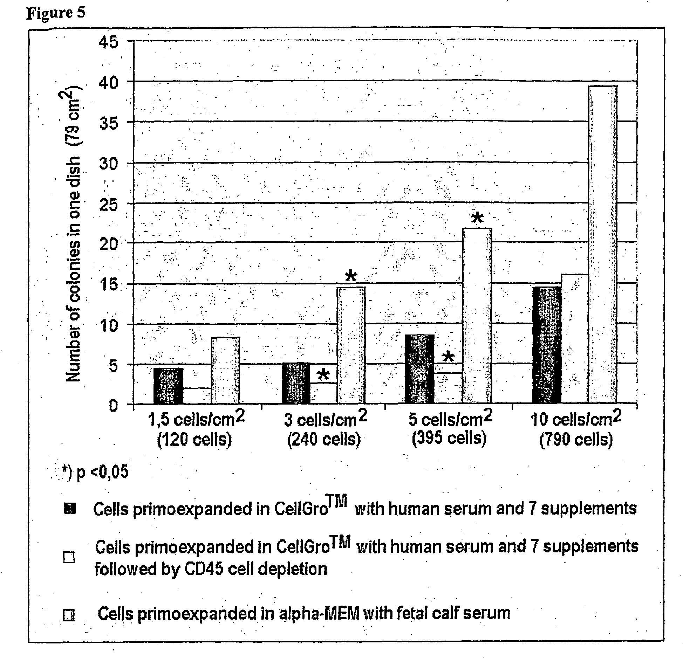

As we have observed a significant increase in the adherent cells yield after the M-CSF and FGF-2 addition to the five basic supplements, we consider the combination of the five basic supplements with these two growth factors in any ratios and concentrations and in any combination with other factors to be the essential feature of our invention. The significance of the sequential addition of the supplements is shown in Example 3 and in Figure 5.

In the first series of experiments, we have used the media formulations a, b and c for the cultivation of mesenchymal stem cells to determine the significance of the sequential addition of the supplements to the medium with human serum. After confirmation of a significant increase of the yield after the addition of M-CSF and FGF-2 to the five basic supplements, in the second series of experiments, we have replaced the commonly used alpha-MEM with the clinical-grade CellGro™ Hematopoietic Stem Cell Medium with the following objectives:

i. to determine whether the addition of the supplements to the clinical grade medium is sufficient to omit the serum,
ii. to evaluate the influence of the change of the medium on the yield of the adherent cells, while the serum remains the same.

The mesenchymal stem cells were cultivated in the above-mentioned seeding concentrations and in the above-mentioned complete media formulations for a two week period under standard conditions (in the incubator with 5% $CO_2$ and at the temperature 37 ˚C). The two week period was chosen arbitrarily to allow for comparison of the results of different experiments. With regard to the fact that in some experiments, better results could be obtained after a longer cultivation period, we do not consider the 14 day cultivation period to be the only possible period. Photographic documentation of the cultures was routinely carried out in days 4, 8 and 14, whereas the seeding day was day 1.

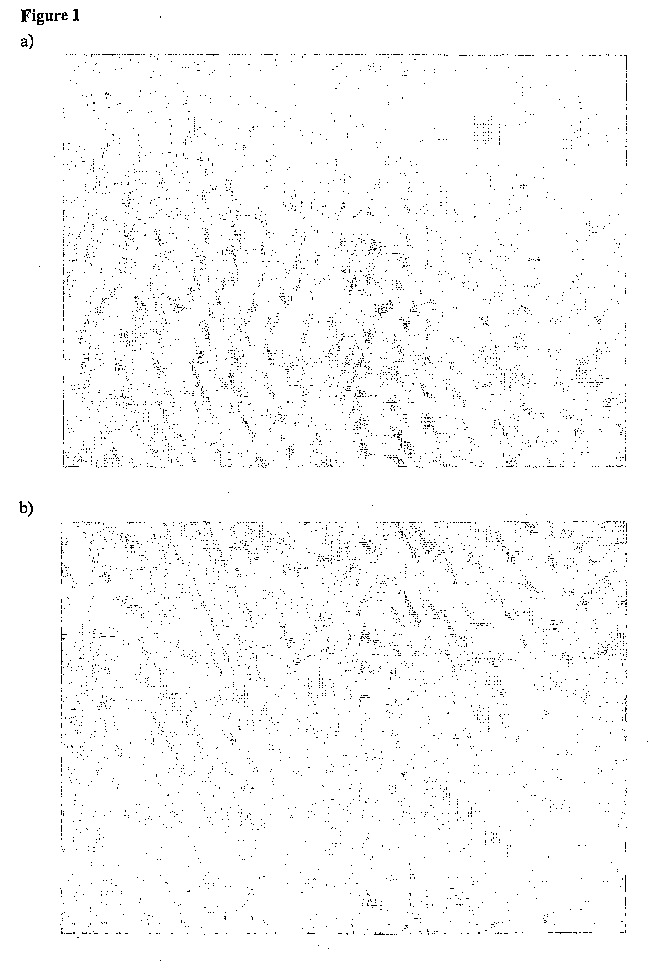

In the cultures with fetal calf serum, the non-adherent cells were removed after 24 hours by rinsing the cultivation vessel with phosphate buffered saline and the adherent cells were fed with fresh complete medium. In these cultures, the culture medium was changed once a week, as in our experience the weekly medium exchange did not lead to the cell starvation or worse yields compared to the twice a week medium exchange. The growth of the mesenchymal stem cells after the removal of the non-adherent cells in the first and the second week of the cultivation is shown in Figure 1.

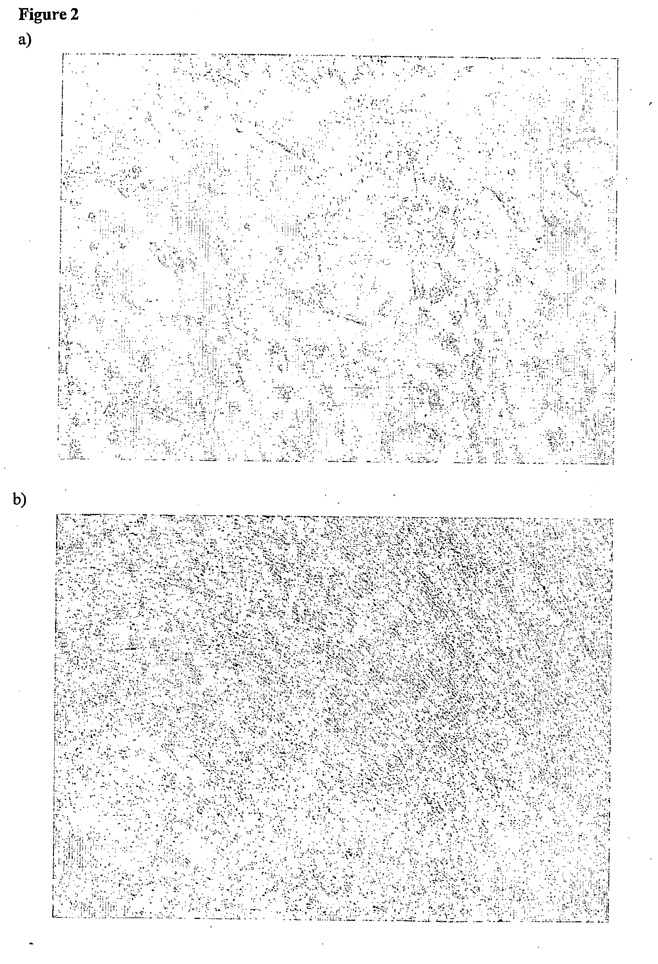

In the cultures with the human serum, our initial experiments have shown that the non-adherent cells need not to be removed and the cultivation medium may not be changed for the whole two week cultivation period. This finding was not published before. The supplements were added twice a week, i.e. at the beginning of the culture and then three times during the two-week cultivation procedure. The non-removal of the non-adherent mononuclear cells and lack of exchange of the cultivation medium did not lead to any starvation of the adherent cells. On the contrary, there was a significant growth acceleration during the second week of the culture (see Fig. 2). The simultaneous cultivation of the non-adherent cells and the adherent cells in the medium with human serum and the supplements without the exchange of the medium during the cultivation period is the essential feature of the present invention.

On day 15 of the culture (initiation of the culture being day 1), the non-adherent cells were removed together with the culture medium, if necessary. The adherent cells were rinsed with the phosphate-buffered saline and harvested with 0.25% EDTA with 1% trypsin solution. After the centrifugation and resuspension in 2 ml of fresh culture medium, the yield of the adherent cells was measured on standard hematological analysers (Beckman-Coulter JT or Beckman-Coulter AcTdiff2, Fullerton, California, USA).

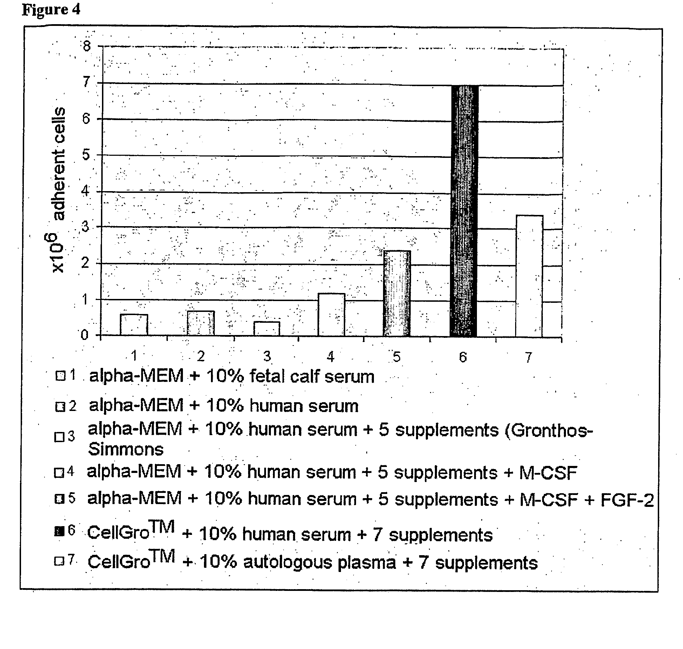

The effect of the use of the CellGro™ Hematopoietic Stem Cell Media without the human serum and the supplements and with the human serum and the supplements is shown in Example 3 and Fig. 4.

4. Determination of the yield of the CD45[neg] CD235a[neg] cells

The mesenchymal stem cells bear on their surface neither the panleukocyte antigen CD45, nor the erythroid cell antigen (CD235a or glycophorin A). As the cultivation of the adherent cells without the removal of the non-adherent cells could lead to a significant contamination with hematopoietic cells, we have taken an advantage of the absence of these antigens and defined the mesenchymal stem cells as the CD45[neg] CD235a[neg] cells in flow cytometry measurements. We have used the monoclonal antibodies CD45 PE-Cy5 and CD235a PE (DakoCytomation, Brno, Czech Republic).

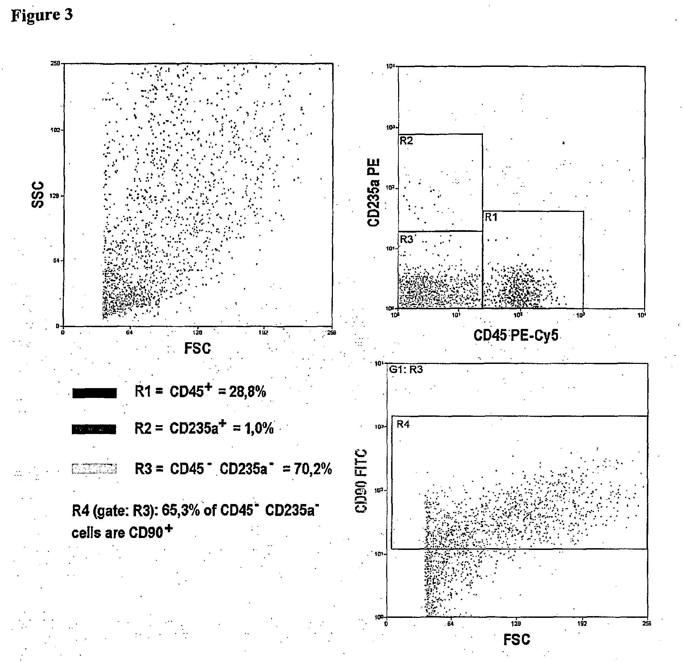

The mesenchymal cells have many antigens (usually adhesive molecules) on their surface, but none of them is specific for these cells. We have therefore alternatively used the monoclonal antibodies against CD29, CD44 or CD90 together with the monoclonal antibodies against CD45 and CD235a, with the aim to determine which antigen is best expressed by the mesenchymal cells grown in different sera. These antibodies were FITC-conjugated. The positivity for any of these antigens was not required for the identification of a particular cell as the mesenchymal cell. The scheme of the calculation of the percentage of the mesenchymal cells in the adherent cell population is shown in Figure 3.

Flow cytometry was performed on the FACSCalibur machine (BD Biosciences Immunocytometry Systems, San Jose, CA) and the yield of marrow stromal cells was determined as:

$$\text{mesenchymal cells count} = \frac{(\text{overall cells count}) \times (\% \, CD45^{neg} \, CD235a^{neg} \, \text{cells})}{100}$$

5. Detailed flow cytometry characterization of mesenchymal cells

Detailed flow cytometry characterization of the mesenchymal cells was performed to search for phenotypic differences between the cells cultivated in alpha-MEM with fetal calf serum and in CellGro™ for Hematopoietic Stem Cells with human serum and the supplements. For this purpose, the adherent cells were depleted of the CD45 positive cells with anti-CD45 immunomagnetic particles on MiniMACS or MidiMACS devices (Milténiy Biotec, Bergish Gladbach, Germany). The flow cytometry was performed on FACSCalibur instrument with the following panel of antibodies: CD11b FITC (BD Biosciences Pharmingen, Erembodegem, Belgie), CD11c FITC (DakoCytomation, Brno, Czech Republic) CD14 FITC or PE (DakoCytomation), CD18 PE (Pharmingen), CD29 PE (Pharmingen), CD34 PE (DakoCytomation), CD44 FITC (Pharmingen), CD45 FITC or PE (DakoCytomation), CD49a FITC (DalcoCytomation), CD49c FITC (R&D Systems, Minneapolis, Minnesotta, USA), CD49d FITC (DakoCytomation), CD49e FITC (R&D Systems), CD63 PE (DakoCytomation), CD71 FITC (DakoCytomation), CD90 FITC (Pharmingen), CD105 FITC and PE (DakoCytomation), CD106 PE (Pharmingen), CD 117 PE (Pharmingen), CD 166 PE (Pharmingen), biotinylated anti ALP (R&D Systems), CXCR4 PE (R&D Systems), anti HLA-A, B, C (DakoCytomation) and anti HLA-DR, DP, DQ (DakoCytomation). Streptavidin-PE for the visualization of the binding of alkaline phosphatase on the cell surface was obtained from Sigma-Aldrich (Steinheim, Federal Republic of Germany). Isotype controls $IgG_1$ FITC, $IgG_1$ PE, $IgG_{2b}$ FITC, $IgG_{2b}$ PE and $IgG_1$ PE-Cy5 were all obtained from DakoCytomation. The results of the detailed immunophenotypizations are shown in Example 4.

6. Secondary colony-formation ability of the mesenchymal stem cells from the primary expansion

Mesenchymal stem cells obtained by the primary expansion in alpha-MEM with fetal calf serum or in the CellGro™ Hematopoietic Stem Cell Medium with human serum and the supplements were reseeded in the densities of 1.5, 3, 5 or 10 cells/cm$^2$ into 100 mm Petri dishes in 10 ml alpha-MEM with fetal calf serum. In some of the experiments, the cells primoexpanded in CellGro™ Hematopoietic Stem Cell Medium with human serum and the supplements were depleted of hematopoietic cells by the anti-CD45 immunomagnetic depletion, as described above. The cells were cultivated for two weeks with the exchange of the medium after the first week. On day 15 (initiation of the culture being day 1), the culture medium was removed, the Petri dishes were rinsed with phosphate-buffered saline and fixed and stained with crystal violet solution. The mesenchymal cell clusters at least 2 mm in diameter were counted by eye as fibroblast colony forming units (CFU-F).

7. Determination of osteogenic potential of mesenchymal stem cells

a. two-dimensional cultures

The mesenchymal stem cells primoexpanded in alpha-MEM with fetal calf serum or in the CellGro™ Hematopoietic Stem Cell Medium with human serum and the supplements were seeded in the concentrations from 10$^3$ to 10$^4$ cells/cm$^2$ into the six-well cultivation plates and cultivated either in the control medium (alpha-MEM + 10% fetal calf serum) or in the osteogenic induction medium (alpha-MEM + 10% fetal calf serum + 10 mM β-glycerol phosphate, 0.1 μM dexamethasone and 0.5 mM ascorbic acid phosphate). The medium was changed once a week for 3-4 weeks. In some experiments, the cells primoexpanded in CellGro™ Hematopoietic Stem Cell Medium with human serum and the supplements were immunomagnetically depleted of the CD45 positive hematopoietic cells. In other experiments, adipogenic differentiation medium was used to determine the potential of the primoexpanded cells for the differentiation into multiple lineages, (induction medium: alpha-MEM + 10% fetal calf serum + 1 μM dexamethasone, 0.2 mM indomethacine, 0.01 mg/ml insulin and 0.5 mM 3-isobutyl-1-methylxanthin; maintenance medium: alpha-MEM + 10% fetal calf serum + 0.01 mg/ml insulin). The induction and differentiation medium were rotated twice a week. All supplements were obtained from Sigma-Aldrich.

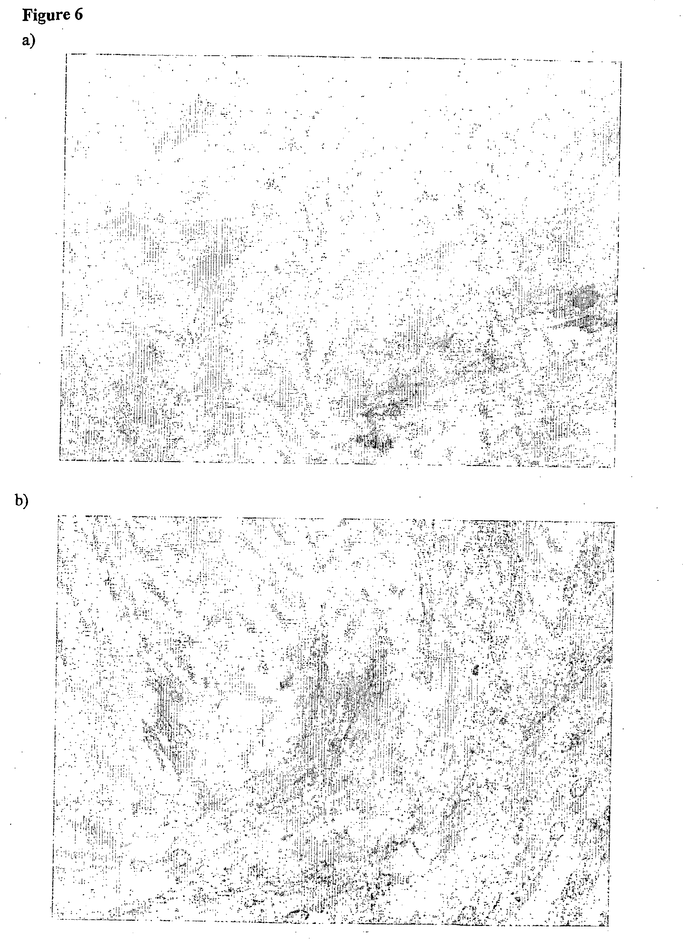

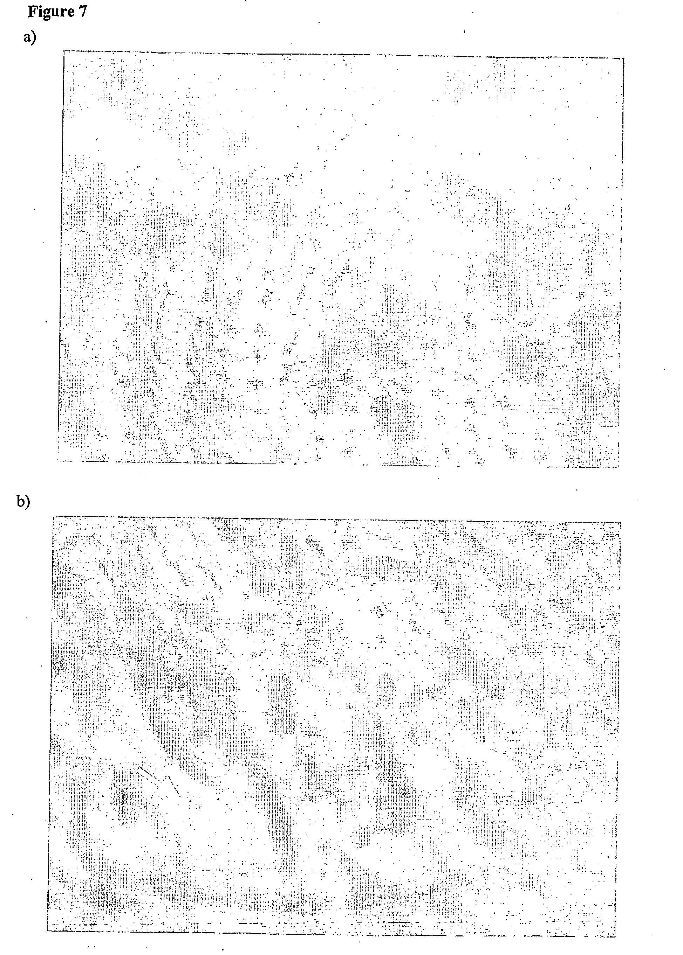

After 3-4 weeks, the cell culture media were discarded and the wells were rinsed with phosphate buffered saline. The osteogenic cultures were either fixed with 4% paraformaldehyde and then stained for alkaline phosphatase with the Alkaline Phosphatase Staining Kit (86-C, Sigma-Aldrich) according to the manufacturer's instruction, or fixed with 2% paraformaldehyde and 0.2% glutaraldehyde and stained by von Kossa staining for osteogenic nodules. Adipogenic cultures were fixed with 70% methanol, stained with oil red and in certain cases co-stained with Mayer's haematoxylin. The results of the osteogenic differentiation in two-dimensional cultures are shown in Example 6 and in Figures 6 and 7.

b. preparation of three-dimensional polylactide scaffolds and the cultivation of the cells on these scaffolds.

Three-dimensional polymeric scaffolds with continuous pores were prepared from poly(L-lactide) fibres. For the preparation of these fibres, high-molecular weight poly(L-lactide) (PLLA) was synthesized. For obtaining good-quality fibres, it was necessary to achieve the mean molecular weight of the polymer of at least 200 000 - 250 000. The PLLA was prepared by the ring-opening polymerization of L-lactide (L-LA) in the melt at the temperature of 110 °C using the stannum(II) 2-ethylhexanoate ($Sn(Oct)_2$) as a catalyst. For achieving the high molecular weight, it is necessary to prepare the monomer (L-lactide) in high purity and to prevent the contact with the air moisture and the contact with porotic materials and surfaces during the manipulation with all components of the polymerization mixture. A typical example of the preparation of the high-molecular polylactide is given as follows:

The monomeric L-lactide (3S-cis-3,6-dimethyl-1,4-dioxan-2,5-dione, Sigma-Aldrich) was repeatedly crystallized from the mixture of dry solvents ethyl acetate/toluene before the use and the crystalline monomer was dried in vacuum. The inner surface of a glass polymerization vessel was silanized by the reaction with dimethylchlorosilane, the vessel was rinsed with hexane and dried in vacuum under anneal. The vessel was filled with 25 g of crystalline L-lactide and 370 $\mu$l of 0.1 M solution of $Sn(Oct)_2$ in anhydrous toluene under inert atmosphere. The mixture was dried at 90 °C in vacuum and the vessel was sealed. The polymerization was performed for 60 hours at the temperature of 110 °C. The polymer was then dissolved in 1 L of dichloromethane and isolated by precipitation in 9 L of methanol. After the filtration, the polymer was dried in vacuum at the temperature of 40 °C. 23.2 g (93 %) of the polymer of mean molecular weight $M_w$ = 360 000 was obtained (determined by the SEC method in tetrahydrofurane).

The polylactide fibres were prepared by the extrusion of the PLLA solution in dichloromethane at a constant speed with a nozzle of circular crosscut and the diameter from 0.6 to 1.5 mm into the coagulation bath (methanol). Using different concentrations of PLLA solution (2-12 % wt.) enabled us to obtain fibres with the diameter from 30-320 $\mu$m. The fibres were rinsed with methanol and dried at room temperature.

The three-dimensional, porous polymer scaffolds were compressed from the PLLA fibres in a mold, which defines the resulting shape and geometric parameters of the scaffold. The fixation of shape and volume of the porous structure was achieved by partial sintering of the fibres in the solvent vapours or by their conglutination at the contact points with the poly(D,L-lactide) solution (PDLLA). Poly(D,L-lactide) ($M_w$ = 630 000) was prepared by the polymerization of D,L-lactide by a procedure similar to that described above for PLLA.

The PLLA was used for the preparation of the fibres because of its good mechanical properties and also because of its limited solubility in solvents such as acetone, THF or toluene. This enables covering the inner surface of the porous structure of the PLLA with a thin layer of PDLLA using the solution of PDLLA in acetone, without the risk of breaking down the porous structure of PLLA. The PDLLA coating on the surface of the fibres strengthens the 3-D structure and forms bridges between the fibres at the sites of their crossing, which produces the continuous surface, necessary for the cell migration into the scaffold. The volume of the pores in the matrix can be adjusted by the density of the fibres and by the rate of their compression. The ratios of the pore volume, the mean diameter of the pores (mean distance between the fibres) and the inner surface of the structure, are dependent not only on the fibre density but also on their diameter.

c. elemental analysis of the extracellular matrix

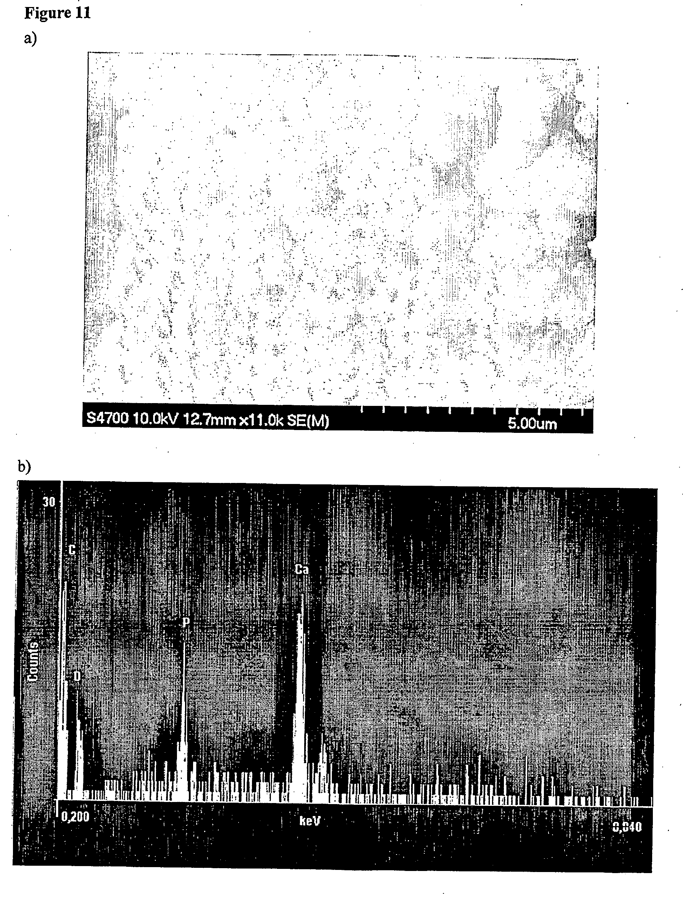

Elemental analysis was performed on the scanning electron microscope Hitachi at the energy of 8,8 keV. The results of the elemental analysis are shown in Figure 11.

d. implantation of the polylactide scaffolds with human mesenchymal cells subcutaneously into immunodeficient mice

The cells were first cultivated on the polylactide scaffolds for 2-4 weeks in osteogenic induction medium either with fetal calf serum or with human serum and the supplements. Immunodeficient (NOD/LtSz-*Rag1null*) mice were

anesthetized by xylazine and marked by fuchsine for further identification. Under aseptic conditions, two cuts on both lateral sides of the thorax were performed and the PLLA scaffolds with the human cells were implanted into the tunnels preformed by blunted preparation. The skin was sutured by standard surgical sewing material and the mice were put back into their hutches. The mice were nursed under the conditions usual for immunodeficient animals, they received sterilized food and sterilized water with antibiotics and were controlled at least every other day. Once in three weeks the mice were anesthetized with ketamine and xylazine and x-rayed under mammograph. An x-ray of one of the mice eight weeks after the implantation of one scaffold with the cells primoexpanded in CellGro™ Hematopoietic Stem Cell medium with human serum and the supplements and one control scaffold without cells is shown in Figure 11b.

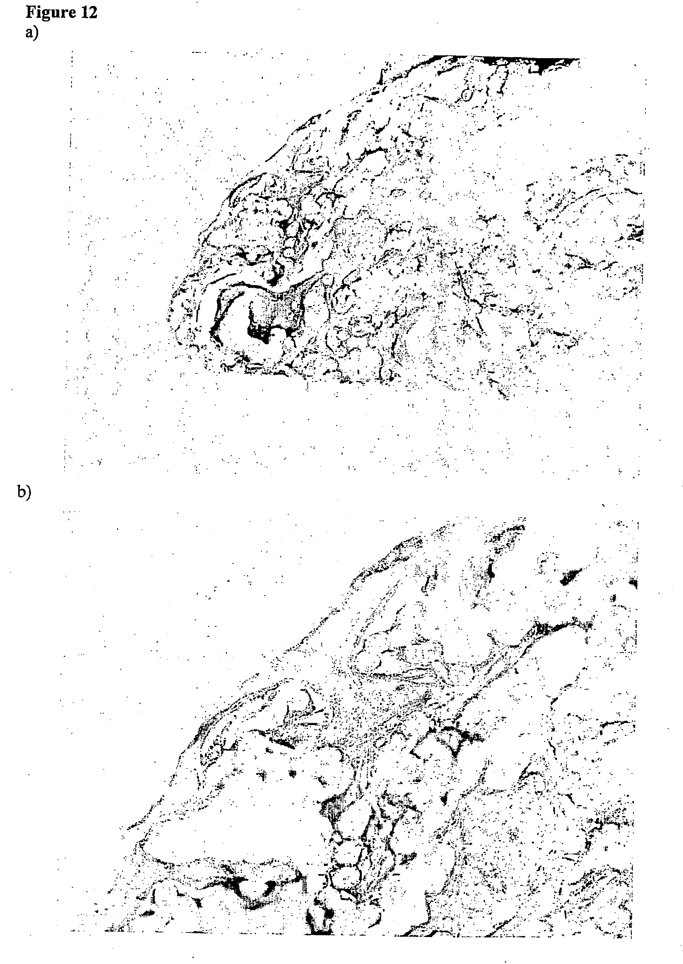

After 6-9 weeks, the mice were sacrificed and immediately after sacrificing fixed by perfusion with 10% formaldehyde. The polylactide scaffolds were removed, transferred into a vessel with 10% formaldehyde and sent for histological and immunohistochemical examination. The results of these examinations are shown in Figure 12. All manipulations with the laboratory animals were performed according to the institutional guidelines for manipulation with experimental animals by the investigators educated and approved for the manipulation with these animals and the research protocol was approved by the Review board for experiments with laboratory animals of the 1st Faculty of Medicine, Charles University, Prague.

8. Principles of the cultivation of adherent cells in closed system

If the mononuclear marrow cells obtained in the suspension should be further manipulated in a closed system, it is necessary to use suitable apparatus enabling the cultivation of the cells in such a system, i.e. a bioreactor. A common cultivation flask is a vessel made of a tissue-treated plastic, i.e. from a plastic the surface of which is treated for optimal spreading and growth of adherent cells, it has walls narrowing to its neck, the neck being equipped with a screw cap with a filter or without it. The cells are grown in these flasks in incubators, usually under the so-called standard conditions (atmosphere with 5% $CO_2$ and the temperature 37 ˚C). The antimicrobial environment in the cultivation vessel and in the incubator is maintained by various methods - either the inner walls of the incubator are coated with copper sheets, or at least the water in the moisturizing vessel contains copper sulphate, or antibiotics and antimycotics may be added into the cultivation medium. The security of the culture can be enhanced by using flasks instead of plastic dishes and by using a screw cap with a filter instead of the cap without a filter, which is not fully closed, so that the exchange of gas between the atmosphere in the flask and the atmosphere in the incubator can be retained. The manipulation with the cultivation vessels is performed in biohazard boxes (with several degrees of Biohazard defined by the number of particles in 1 $m^3$ of air, wherein the Biohazard III degree is considered necessary for the manipulation with clinical grade tissues). During the manipulation with the vessel inside the biohazard box, the cap has to be unscrewed or the cover of the cultivation dish must be removed, which by itself means a further risk of contamination. The greatest risk of contamination is associated with the medium exchange and especially with the passaging of the cells, which means transferring of the adherent cells into the suspension (also called cell harvest), which then is subsequently centrifuged or processed outside the biohazard box. Thus, the single-step cultivation method of the present invention without the passaging step avoids this contamination risk.

Another risk is the opening of the cultivation vessel in the biohazard box. Although the contamination risk is substantially lowered in the biohazard box by the streamline flow of filtered air and by observing the working instructions, this risk still exists (e.g. when pipetting solutions, whereby in the air streamline flow the aerosol can be formed). However, this risk can be avoided by means of a silicon rubber or plastic septum, which is used e.g. in transfusion bags and infusion kits. The septum can be repeatedly penetrated with an injection needle, so that the solutions can be added or removed. Equipping the cultivation vessel with a septum would bring the possibility to continue in the cultivation of the cells in the closed system also after their processing as described above. Such a vessel would become a logical part of the cultivation system. The use of airtight silicon septa would constitute the necessity to fit the upper side of the vessel with a filter. The use of the septa and the application of the cell suspension, media and supplements through the septa would be advantageous only when it is not necessary to change repeatedly the whole content of the cultivation vessel. In case of the method of the present invention, between the step of filling the vessel and the step of removing cells it is only necessary to add the supplements three times by an extra-thin syringe (e.g. orange 25 G 1", Braun). Nevertheless, during a one-shot addition of a larger volume of a solution or during the terminal removal of the cell suspension, undesired overpressure or underpressure could occur, which could not be compensated by the filters. Therefore, we propose that the cultivation vessel is preferably equipped with at least two inlets, whereas when a solution is added through one inlet, the overpressure could be relieved by sucking the gas through a second inlet, and on the other hand, adding the gas with a syringe equipped with an injection needle could facilitate harvesting the cell suspension. Adding or removing the content of the vessels by sterile clinical grade injection needle and syringe should be carried out in the biohazard box of sufficient biohazard grade.

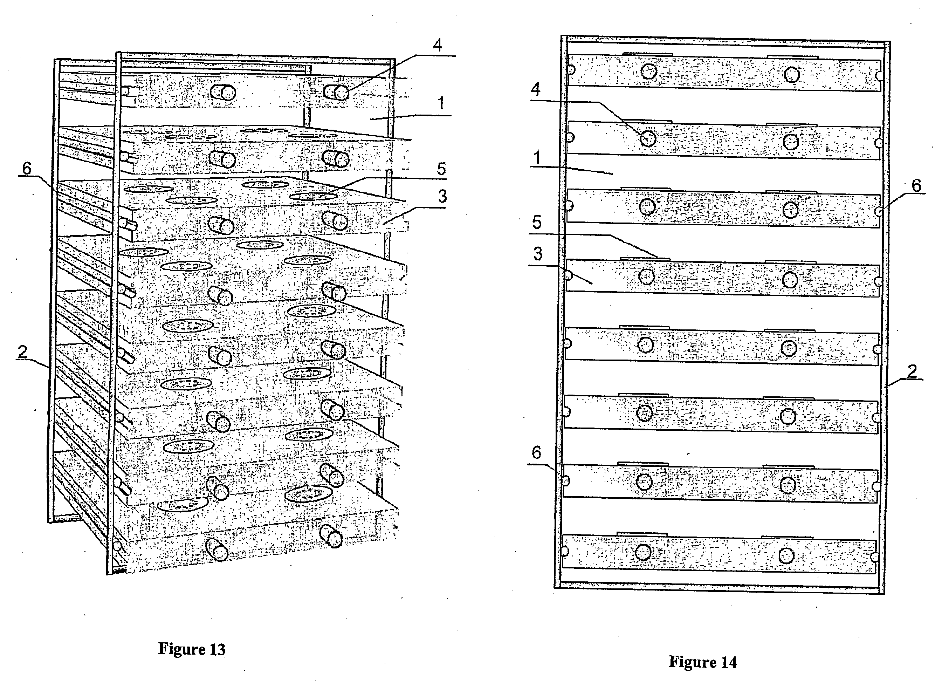

For securing sterile conditions during the cultivation procedure, it would be ideal to have a specialized incubator, in which the vessels would be ordered as a cassette system and which would be used solely for this purpose. Such an apparatus would be quite costly and its construction exceeds the knowledge of the inventors. The inventors consider that any conventional clinical grade $CO_2$ incubator with inserted bioreactor consisting of the carrier and the vessels can be used (see example 10 and Fig. 13 to 20).

Example 1 - Aseptic bone marrow mononuclear cell harvest

In the years 2004-2005, the aseptic bone marrow mononuclear cell harvest was performed in 15 patients suffering from peripheral artery occlusive disease as described above (point 2). The age of the patients was in the range of 26-85 years, two of them were harvested twice. From the mononuclear concentrate, the samples for the mesenchymal cell cultivation were obtained and the remaining cells were applied by intra-arterial infusion to the ischemic leg in the context of experimental protocol of the cellular treatment of peripheral artery occlusive disease. Harvest characteristics are shown in Table 1, while the descriptive statistics of the results for the whole cohort of patients is shown in Table 2.

The results shown below implicate that for the inoculation of one cultivation vessel having the surface of the bottom 75 $cm^2$ with $2.5 \times 10^6$ mononuclear cells, the mean volume of only 23 $\mu l$ of cellular suspension was necessary (range, 11-69 $\mu l$). This further implicates that for obtaining a sufficient number of mononuclear cells, substantially less marrow blood would be needed than was harvested in our patients. 20-30 ml of marrow blood would be enough for obtaining sufficient number of CFU-F in all instances (see Examples 3 and 5).

Table 1

| Patient initials | Age | Sex | Volume of marrow blood harvested (ml) | Final volume of marrow mononuclear cell suspension (ml) | Total number of marrow mononuclear cells obtained ($\times 10^8$) | Concentration of marrow mononuclear cells in 1 ml of final suspension ($\times 10^6$) | Volume of mononuclear suspension necessary to obtain $2.5 \times 10^6$ cells (in $\mu l$) |
|---|---|---|---|---|---|---|---|
| K.A. | 70 | female | 436 | 56 | 51.13 | 91.3 | 27 |
| K.A. | 76 | male | 323 | 50 | 18.13 | 36.3 | 69 |
| O.K. | 26 | male | 600 | 36 | 79.2 | 220 | 12 |
| K.L. | 40 | male | 700 | 40 | 94 | 235 | 11 |
| M.M. | 75 | female | 540 | 42 | 57.22 | 136.2 | 18 |
| L.J. | 43 | male | 400 | 45 | 69.6 | 154.7 | 16 |
| A.J. | 61 | male | 400 | 35 | 45.5 | 130 | 19 |
| C.E. | 39 | female | 400 | 50 | 55 | 110 | 23 |
| S.V. | 73 | male | 643 | 53 | 45.6 | 91.2 | 28 |
| B.E. | 47 | female | 440 | 48 | 65.52 | 136.5 | 19 |
| H.J. | 85 | male | 450 | 25 | 23.8 | 95.2 | 27 |
| S.V. | 68 | male | 600 | 35 | 63.67 | 209 | 12 |
| C.E. | 39 | female | 366 | 42 | 46.2 | 110 | 23 |
| Z.M. | 67 | female | 455 | 49 | 61.25 | 125 | 20 |
| M.J. | 52 | female | 346 | 44 | 37.4 | 85 | 30 |
| H.A. | 75 | female | 350 | 74 | 125.8 | 170 | 15 |
| L.J. | 43 | male | 440 | 68 | 95.2 | 140 | 18 |

Table 2

| | |
|---|---|
| Number of patients | 15 |
| Men: women (ratio, %) | 8 : 7 (53% : 47%) |
| Number of harvests | 17 |
| Volume of the marrow blood harvested (ml, median and range) | 440 (323 - 700) |
| Final volume of the mononuclear suspension (ml, median and range) | 45 (25-74) |

(continued)

| | |
|---|---|
| Number of mononuclear cells obtained (x$10^8$, median and range) | 57,2 (18,1-125,8) |
| Concentration of mononuclear cells in the final suspension($10^6$/ml, median and range) | 130 (36-235) |
| Volume of the mononuclear concentrate containing 2,5x$10^6$ cells (median and range) | 19 µl (11- 69 µl) |

Example 2 - Cultivation of the cells in CellGro™ Hematopoietic Stem Cell Medium with human serum and the supplements

In 2005, mononuclear bone marrow cells from 18 research subjects were cultivated in CellGro™ Hematopoietic Stem Cell Medium with human serum and the supplements. This cohort is somehow different from the cohort described in Example 1, because certain samples from patients characterized in Example 1 were not cultivated in this medium (the medium was not available at that time). To make the cohort larger and more representative, it was later completed with patients who underwent the bone marrow harvest from diagnostic or follow-up purposes for suspected or established haematological disease. Demographic characteristics of the subjects, whose cells were grown in CellGro™ Hematopoietic Stem Cell Medium with human serum and the supplements, are shown in Table 3 and the cultivation results in Table 4.

Table 3

| | |
|---|---|
| Age (median and range) | 65,5 years (39-78 year) |
| Men : women (ratio, percentages) | 7 : 11 (39% v. 61%) |
| Patients with peripheral artery occlusive disease or healthy subjects v. patients with haematological disease (numbers, percentages) | 8:10 (44: 56%) |
| Patients with bone marrow infiltrated by haematological disease v. patients without bone marrow infiltration (number, percentages) | 5 : 13 (27% v. 73%) |
| CellGro™ medium batch used (1 v. 2, numbers, percentages) | 7: 11 (39% v 69%) |

Table 4

| | |
|---|---|
| Number of mononuclear cells seeded (in 75 cm$^2$ vessel) | á 2.5x$10^6$ |
| Number of harvested adherent cells (median and range) | 7.0x$10^6$ (1.4-10x$10^6$) |
| Percentage of CD45$^-$ CD235a$^-$ cells (mesenchymal cells, median and range) | 88.2% (63.9% - 96.5%) |
| Total number of CD45$^-$CD235a$^-$ cells (mesenchymal cells, median and range) | 6.19x$10^6$ (1.12-7.88x$10^6$) |

Because the mononuclear cells from the bone marrow of the patients with haematological disease might have had different characteristics from the mononuclear cells of the healthy subjects and the results might have been further influenced by the age and the sex of the patients, the bone marrow involvement with haematological disease and the batch of CellGro™ Hematopoietic Stem Cell Medium (two batches were used), we have performed statistical analysis accounting for these variables. There was very a weak and statistically insignificant negative correlation between the age and the number of harvested adherent cells, the percentage of mesenchymal cells in the harvested adherent cells and the total number of harvested mesenchymal cells. Univariate analysis has not shown any dependence of the number of harvested adherent cells, the percentage of MSC in the adherent cells, or the total number of harvested MSC on the sex, diagnosis, bone marrow involvement with haematological disease or batch of CellGro™ Hematopoietic Stem Cell Medium used.

Example 3 - Comparison of the yields of mesenchymal stem cells cultivated in different media with different combinations of supplements

Comparison of the yields of adherent cells in different media and with different combinations of supplements was performed with the Mann-Whitney U test and Kruskal-Wallis variation of ANOVA. The results are shown in Tables 5 and 6 and in Figure 4. In Table 5, the medians of the yields from the media and the ranges between the 25th and the 75th percentile are shown. P ≤ 0.05 is considered as statistically significant.

Table 5 - Mann-Whitney test, yields of adherent cells from different media with different sera and different supplements

| Complete cultivation medium | Number of experiments | Median (x$10^6$ cells) | 25th to 75th percentile (x$10^6$ cells) | Statistical comparison with CellGro™ + HS + 5S |
|---|---|---|---|---|
| Alpha-MEM + FCS | 35 | 0.6 | 0.4-1.0 | p <0.001 |
| Alpha-MEM+HS | 20 | 0.7 | 0.4-1.1 | p <0.001 |
| Alpha-MEM + HS +5S | 7 | 7 0.4 | 0.25-1.1 | p <0.001 |
| Alpha-MEM + HS + 5S + M-CSF | 5 | 1.2 | 0.55-1.95 | p = 0.002 |
| Alpha-MEM + HS + 5S + M-CSF + FGF2 | 21 | 2.4 | 1.2-5.75 | p <0.001 |
| CellGro™ + HS + 5S +M-CSF + FGF2 | 18 | 7.0 | 5.4-8.4 | |
| CellGro™ + AP + 5S + M-CSF + FGF2 | 5 | 3.4 | 1.55-5.7 | p = 0.136 |

Table 6 - Kruskal-Wallis test with Dunn's comparisons, multivariant analysis

| Comparison | Statistical significance (p ≤ 0.05) |
|---|---|
| CellGro™ + HS + 5S + M-CSF + FGF2 v. alfa-MEM + FCS | yes |
| CellGro™ + HS + 5S + M-CSF + FGF2 v.alfa-MEM + HS | yes |
| CellGro™ + HS + 5S + M-CSF + FGF2 v. alfa-MEM + HS + 5S | yes |
| CellGro™ + HS + 5S + M-CSF + FGF2 v. alfa-MEM + HS + 5S + M-CSF | no |
| CellGro™ + HS + 5S + M-CSF + FGF2 v. alfa-MEM + HS + 5S + M-CSF + FGF2 | cannot be tested |
| CellGro™ + HS + 5S + M-CSF + FGF2 v. CellGro™ + AP + 5S + M-CSF + FGF2 | cannot be tested |
| Abbreviations (in both tables): FCS = fetal calf serum, HS = human serum, AP = autologous plasma, 5S = five supplements according to Gronthos and Simmons (ascorbic acid, dexamethasone, EGF, insulin and PDGF-BB), M-CSF = macrophage colony-stimulating factor, FGF-2 = fibroblast growth factor 2. | |

The results presented above show that the number of harvested adherent cells was highest in the complete medium consisting of CellGro™ Hematopoietic Stem Cell Medium with human serum and the seven supplements (Gronthos-Simmons 5 basal supplements with M-CSF with FGF2) and that the yields of adherent cells from this medium were significantly higher than from any other medium, with the exception of CellGro™ Hematopoietic Stem Cell Medium with autologous plasma and the supplements. The lack of statistical significance in this case as well as the lack of statistical significance in certain comparisons in multivariant analysis may be explained by the fact that these tests had no adequate statistical power for detection of statistical significance due to the low number of samples.

Figure 4 further shows that the yield of adherent cells has grown steadily with the number of supplements added to the alpha-MEM with human serum, that a significant improvement was observed after the addition of M-CSF and FGF2 to the complete medium (alpha-MEM with human serum and five Gronthos-Simmons basal supplements) and that another significant improvement was achieved after the replacement of alpha-MEM with CellGro™ Hematopoietic Stem Cell Medium. The results further show that the replacement of pooled human serum with autologous plasma does not lead to further increase of the yield of adherent cells, but on the contrary, it is possible that in this case, the yield of adherent cells decreases.

Because the adherent fraction contains, apart from the mesenchymal stem cells, also hematopoietic cells, we have performed in a smaller number of samples the measurements of the percentage of mesenchymal cells (i.e., $CD45^{neg}$ $CD235a^{neg}$ cells) in the adherent fraction by flow cytometry and then we have counted the number of mesenchymal cells according to the formula shown in Point 4 above. Because in nine cases these measurements were performed on the samples from the same research subject cultivated in different culture media, we could use the paired t-test for the assessment of the influence of the replacement of alpha-MEM with CellGro™ Hematopoietic Stem Cell Medium. These results are shown in Table 7. Results are shown as means with standard deviation, statistical analysis compares numbers of $CD45^{neg}$ $CD235a^{neg}$ cells.

Table 7 - the influence of the replacement of alpha-MEM with CellGro™ Hematopoietic Stem Cell Medium on the number of harvested $CD45^{neg}$ $CD235a^{neg}$ cells (i.e., mesenchymal stem cells)

| Complete culture medium | Number of adherent cells | Percentage of $CD45^{neg}$ $CD235a^{neg}$ cells | Absolute number of $CD45^{neg}$ $CD235a^{neg}$ cells | Statistical significance |
|---|---|---|---|---|
| alfa-MEM + HS + 5S + M-CSF + FGF2 | $3.69 \pm 2.53 \times 10^6$ | $80.1 \pm 12.4\%$ | $3.06 \pm 2.31 \times 10^6$ | p = 0.019 |
| CellGro™ + HS + 5S+M-CSF+FGF2 | $5.47 \pm 3.14 \times 10^6$ | $85.7 \pm 10.3\%$ | $4.62 \pm 2.96 \times 10^6$ | |

These results show that the replacement of alpha-MEM with CellGro™ Hematopoietic Stem Cell Medium not only leads to a higher number of adherent cells, but also that the adherent cells contain higher percentage of mesenchymal cells, which both leads to a significantly better yield of harvested $CD45^{neg}$ $CD235a^{neg}$ cells.

In general, the present example shows that a significant improvement in the yield of adherent cells was achieved with addition of M-CSF and FGF2 to the five basal supplements according to Gronthos and Simmons, that further improvement of the yield was achieved by the replacement of alpha-MEM with CellGro™ Hematopoietic Stem Cell Medium certified for the clinical use and that with this embodiment of the present invention it is possible to harvest more than $5 \times 10^6$ adherent cells from the originally seeded $2.5 \times 10^6$ marrow mononuclear cells in 75% of research subjects during single expansion. 85% of these cells are double negative for CD45 a CD235a and therefore are compatible with the definition of the mesenchymal stem cells.

Example 4 - Comparison of the immunophenotype of the mesenchymal stem cells expanded in CellGro™ Hematopoietic Stem Cell Medium with human serum and the supplements with the mesenchymal stem cells expanded in alpha-MEM with fetal calf serum.

To investigate how the phenotype of the cells expanded in CellGro™ Hematopoietic Stem Cell Medium with human serum and the supplements differs from the phenotype of the cells expanded in standard alpha-MEM medium with fetal calf serum, we have characterized the surface markers by flow-cytometric measurement. Positivity for a given marker was expressed as the percentage of cells with brighter fluorescence than was the fluorescence of 0.5% of the brightest negative control cells. The negative control cells consisted of the cells from the same harvest stained with an irrelevant isotypic immunoglobulin with an appropriate fluorescent compound. Alkaline phosphatase (ALP) positivity was determined with biotinylated anti-ALP antibody, on which in the second step streptavidin with phycoerythein was conjugated. Negative control in this case were cells incubated with phycoerythein conjugate only, while the biotinylated antibody was omitted.

Before the flow-cytometric measurements were carried out, the adherent cells grown in the CellGro™ Medium were depleted of hematopoietic cells by incubation with anti-CD45 immunomagnetic particles and subsequent removal of CD45-positive cells by means of the magnetic apparatus MiniMACS or CliniMACS (Miltényi Biotec, Germany).

Differences in the positivity of the cells expanded in CellGro™ Hematopoietic Stem Cell Medium with human serum and the supplements and the cells grown in alpha-MEM medium with fetal calf serum for individual surface markers were compared statistically with t-test and Mann-Whitney U test. The results are expressed in Table 8 as medians and statistical significance values p from the Mann-Whitney U test. The results of the t-test were similar, but we could not use the paired t-test or its non-parametric variant because of the low number of paired samples from the same individuals. $P \leq 0.05$ was considered as statistically significant.

Table 8

| | alfa-MEM + FCS | | CellGro™ + HS + supplements | | |
|---|---|---|---|---|---|
| Marker | Number of samples | Positive cells, median (%) | Number of samples | Positive cells, median (%) | Statistical significance |
| CD 11c | 13 | 26.3% | 6 | 11.1% | p = 0.048 |
| CD 29 | 9 | 77.6% | 7 | 95.0% | p <0.001 |
| CD 44 | 9 | 52.2% | 7 | 90.8% | p = 0.011 |
| CD 45 | 8 | 15.2% | 7 | 8.8% | p = 0.152 |
| CD 49a | 9 | 14.2% | 7 | 14.3% | p = 0.953 |
| CD 49c | 9 | 6.3% | 7 | 34.7% | p = 0.001 |
| CD 49d | 9 | 9.3% | 7 | 30.2% | p = 0.008 |
| CD 49e | 9 | 15.1% | 7 | 25.8% | p = 0.244 |
| CD 63 | 9 | 55.6% | 7 | 64.2% | p = 0.244 |
| CD 71 | 9 | 21.6% | 7 | 47.4% | p = 0.034 |
| CD 90 | 6 | 58.1% | 7 | 65.0% | p = 0.731 |
| CD 105 | 9 | 44.2% | 7 | 25.5% | p = 0.290 |
| CD 106 | 8 | 3.7% | 7 | 13% | p = 0.014 |
| CD 166 | 8 | 50.8% | 7 | 31.0% | p = 0.072 |
| ALP | 7 | 15.1 % | 7 | 14.2% | p =1.000 |
| Abbreviations: FCS = fetal calf serum, HS = human serum. | | | | | |

The results show that due to the partial CD45+ cell depletion from the adherent cells grown in CellGro™ Hematopoietic Stem Cell Medium with human serum and the supplements, the contamination of the mesenchymal cells with hematopoietic cells is similar in both groups of samples. Therefore, the results of the measurements should not be affected by different level of contamination with the hematopoietic cells. The mesenchymal cells are characterized, besides their differentiation abilities, also by certain surface markers, among them by the expression of CD29, CD44, CD63, CD90, CD105, CD106 a CD166. The results of the measurements show that the mesenchymal cells primoexpanded in CellGro™ Hematopoietic Stem Cell Medium bear these molecules in a similar amount (CD63, CD90, CD105, CD166) or a higher amount (CD44, CD29, CD 106) than their counterparts primoexpanded in alpha-MEM with fetal calf serum, and thus fulfil the phenotypic characteristics of mesenchymal stem cells. The marker CD71 is the marker of growth activity and this marker is more expressed in the mesenchymal cells primo expanded in CellGro™ Hematopoietic Stem Cell Medium with human serum and the supplements. Thus, the cells expanded in CellGro™ Hematopoietic Stem Cell Medium with human serum and the supplements possess a higher growth activity than the cells expanded in alpha-MEM with fetal calf serum. This is in agreement with the fact that higher yields of mesenchymal cells were observed in CellGro™ Hematopoietic Stem Cell Medium. The expression of alkaline phosphatase (ALP) shows how many mesenchymal cells already in the course of the primary expansion differentiate into the osteoblastic lineage. The percentage of the cells positive for alkaline phosphatase is comparable in cells grown in either media.

This example shows that the adherent non-hematopoietic stem cells cultivated from the mononuclear bone marrow cells in CellGro™ Hematopoietic Stem Cell Medium with human serum and the supplements have the phenotype characteristics of mesenchymal stem cells at least comparable with the cells cultivated in alpha-MEM with fetal calf serum, they have a good growth activity and part of them differentiates into the osteoblastic lineage even during the primary expansion.

Example 5 - Comparison of the secondary CFU-F formation from the mesenchymal stem cells primoexpanded in alpha-MEM medium with fetal calf serum and in CellGro™ Hematopoietic Stem Cell Medium with human serum and the supplements and calculation of the number of CFU-F units obtained during the primary expansion

It is widely accepted, that not every mesenchymal stem cell is clonogenic, i.e. able of further division. The clonogenicity of the cells is studied by the CFU-F assay, as described by Colter et al. (Colter DC, et al: Rapid expansion of recycling stem cells in cultures of plastic-adherent cells from human bone marrow. Proc Natl Acad Sci USA. 2000; 97: 3213-3218) and successfully reproduced in our laboratory *(Novotová E, Strnadová H, Procházka B, a Pytlik R. In vitro kultivace mezenchymových kmenových buněk u pacientů s lymfoidními malignitami. Transfuse dnes, 2003; 9: 28-34).* The principle of the CFU-F assay is briefly described above in Point 6. Using this assay, we have evaluated the possibility to obtain sufficient number of GFU-F needed for the healing of bone fracture. According to the work of Hernigou, recited herein above, we have assumed that approximately 50 000 CFU-F is needed for successful treatment of the fracture *(*Hernigou P, et al: Percutaneous autologous bone-marrow grafting for nonunions. J Bone Joint Surg Am. 2005; 87: 1430-1437*).*

By the method described herein above in Point 6, the cells from seven research subjects, primo expanded either in alpha-MEM with fetal calf serum or in CellGro™ Hematopoietic Stem Cell Medium with human serum and the supplements, were cultivated. For determining whether the number of CFU-F is affected by the presence of hematopoietic cells, we have performed the CD45 cell depletion in some samples of adherent cells obtained from CellGro™ Hematopoietic Stem Cell Medium with human serum and the supplements, using the antiCD45 antibody with immunomagnetic particles on the MiniMACS (Miltényi Biotec). The number of secondary colonies was compared by paired t-test. The results of these experiments are shown in Tables 9 and 10 and in Figure 5.

Table 9

| Number of secondary CFU-F in dependence on the method of primary expansion and secondary seeding concentration in a 79 cm$^2$ dish | | | | |
|---|---|---|---|---|
| | Number of secondary colonies after secondary seeding | | | |
| Method of primary expansion | 1.5 cell/cm$^2$ (120 cells) | 3 cells/cm$^2$ (240 cells) | 5 cells/cm$^2$ (395 cells) | 10 cells/cm$^2$ (790 cells) |
| Alpha-MEM + FCS | 8.3 ± 8.1 | 14.6 ± 10.1 | 21.7 ± 16.2 | 39.4 ± 35.0 |
| CellGro™ + HS + supplements | 4.6 ± 7.6 | 5.1 ± 6.0 | 8.6 ± 8.7 | 14.6 ± 8.6 |
| CellGro™ + HS + supplements with CD45 depletion) | 2.1 ± 3.2 | 2.7 ± 4.2 | 3.9 ± 4.5 | 16.0 ± 20.8 |
| FCS = fetal calf serum, HS = human serum, supplements = five Gronthos basal supplements + M-CSF + FGF-2. | | | | |

Table 10

| Estimate of the number of cells necessary for one CFU-F colony formation | | | | |
|---|---|---|---|---|
| | Number of seeded cells necessary for the formation of one colony | | | |
| Method of primary expansion | 1.5 cell/cm$^2$ (120 cells) | 3 cells/cm$^2$ (240 cells) | 5 cells/cm$^2$ (395 cells) | 10 cells/cm$^2$ (790 cells) |
| Alpha-MEM + FCS | 15 (8 - 600) | 17 (10 - 54) | 19 (11 - 72) | 20 (11 - 180) |
| CellGro™+HS+ supplements | 27 (10 - NPtC) | 47 (22 - NPtC) | 46 (23 - NPtC) | 54 (34 - 132) |
| CellGro™ + HS + supplements (with CD45 depletion) | 57 (23 - NPtC) | 89 (35 - NPtC) | 102 (47 - NPtC) | 50 (26 - NPtC) |
| FCS = fetal calf serum, HS = human serum, supplements = five Gronthos basal supplements + M-CSF + FGF-2, NPtC = not possible to count. | | | | |

From the results shown above it is clear that the cells primo expanded in alpha-MEM with fetal calf serum have better secondary CFU-F formation ability than the cells primo expanded in CellGro™ with human serum and the supplements (even though these result did not reach statistical significance due to the low number of experiments). However, it is necessary to consider that the secondary CFU-F formation was studied in alpha-MEM with fetal calf serum in all cases, i.e. the cells primoexpanded in CellGro™ with human serum and the supplements were cultivated in a different medium than was the medium used for primary expansion and also under different conditions than the *in vivo* conditions. However, even if we assume that the secondary colony-forming ability is really diminished in the mesenchymal cells primoexpanded in CellGro™ Hematopoietic Stem Cell Medium with human serum and the supplements, this is compensated by higher yield of the cells primoexpanded in this medium (see example 3). If we assume that only one of 50 cells obtained by the primary expansion in CellGro™ with human serum and the supplements is clonogenic, it still means that from $5x10^6$ adherent cells obtained, approximately 100 000 colony-forming cells can be obtained, which is, according to Hernigou, sufficient for the acceleration of poor fracture healing. The mentioned number of $5x10^6$ adherent cells can be achieved in more than 75% of cases from as little as $2.5x10^6$ mononuclear bone marrow cells during single expansion, taking approximately two weeks (see Example 3, Table 5).

From the results shown above it can be concluded that the adherent cells, obtained by the primary expansion in CellGro™ Hematopoietic Stem Cell Medium with human serum and the supplements can be used for orthopaedic purposes without the CD45 depletion, because the secondary CFU-F formation without the hematopoietic cell depletion (i.e. from the cells containing lower percentage of mesenchymal cells than the cells after the CD45 depletion) is at least as good as after the CD45 depletion. This fact cannot be explained by the despatch of a large amount of mesenchymal stem cells during the depletion procedure, because the amount of dead cells after the depletion procedure ranged between 3 and 23 %, while the percentage of dead cells among the cells obtained from alfa-MEM with fetal calf serum and the supplements ranged from 13 to 52 % (measured with 7-AAD on a flow cytometer). These results thus confirm the theoretical concept that the contamination of mesenchymal cells by hematopoietic cells is useful not only for their primary expansion, but also for their further growth. From the practical point of view, it means that the mesenchymal cells for orthopaedic purposes do not need to be purified from the hematopoietic cells.

This example therefore implies that by the method described herein above, it is possible to obtain a sufficient number of CFU-F for healing of a poorly-healing fracture during the single expansion from a low initial number of mononuclear cells.

The admixed hematopoietic cells do not have any detrimental effect on the further growth of the mesenchymal cells.

Example 6 - Differentiation of mesenchymal stem cells in two-dimensional cultures.

The mesenchymal cells primoexpanded in CellGro™ Hematopoietic Stem Cell Medium with human serum and the supplements and the mesenchymal cells primoexpanded in alpha-MEM with fetal calf serum were seeded in the densities 1000-2500 cells/cm² in six-well culture plates with the surface of one well of approximately 10 cm² and cultivated in 2 ml of osteogenic induction medium (see above, point 3). The medium was changed once a week and the cell growth and the formation of bone nodules was observed under inverted microscope. After 2-4 weeks of expansion, the cells were either fixed with 4% paraformaldehyde in phosphate buffered saline and then stained for alkaline phosphatase activity with Sigma C86 kit (Sigma-Aldrich, Germany) or fixed with 2% paraformaldehyde with addition of 0.2% glutaraldehyde in phosphate buffered saline and stained by von Kossa staining.

The results are shown in Fig. 6 and 7. The cells primoexpanded in CellGro™ Hematopoietic Stem Cell Medium with human serum and the supplements have formed visible bone nodules after two weeks only (6a), and these nodules were positive for von Kossa staining (6b). In the cells primoexpanded in alpha-MEM with fetal calf serum, this fast formation of visible bone nodules was not observed, though the wells with these cells were also positive for von Kossa staining after four weeks of cultivation. Cells primoexpanded in both culture media showed comparable alkaline phosphatase activity after 3-4 weeks in osteogenic culture (7a, cells primoexpanded in alpha-MEM with fetal calf serum, 7b, cells primoexpanded in CellGro™ Hematopoietic Stem Cell Medium with human serum and the supplements), while the positivity of the cells primoexpanded in CellGro™ Hematopoietic Stem Cell Medium with human serum and the supplements was at least as pronounced as the positivity of the cells primoexpanded in alpha-MEM with fetal calf serum.

This example therefore shows that the cells primoexpanded in CellGro™ Hematopoietic Stem Cell Medium with human serum and the supplements form the bone nodules under osteogenic conditions *in vitro* faster than the cells primoexpanded in alpha-MEM with fetal calf serum and show at least comparable alkaline phosphatase activity, which marks the differentiation towards osteoblastic lineage.

Example 7 - Preparation of polylactide carriers and bone matrix formation by cells expanded in CellGro™ Hematopoietic Stem Cell Medium with 10% human serum and the supplements

By the procedure described herein above, Point 7b, porous polylactide carriers (scaffolds) were prepared from PLLA fibres in the shape of small tablets with the diameter of 5.6 mm, the thickness of 1.5 to 2 mm and the distance between fibres of 100-400 $\mu$m for the cultivation of mesenchymal cells. The tablets were sterilized under germicide (UVB) lamp for 2 hours and subsequently immersed into the osteogenic medium with fetal calf serum or human serum for 3-24 hours before the cell seeding, so that they become soaked with the medium, and the air bubbles were removed.

The cells primoexpanded in alpha-MEM with fetal calf serum or in CellGro™ Hematopoietic Stem Cell medium with human serum and the supplements were then seeded in the amount of $2\times10^5$ of cells per scaffold, while to the osteogenic medium with human serum, 10 ng/ml EGF, 100 ng/ml PDGF-BB, 25 ng/ml M-CSF and 1 ng/ml FGF-2 were added, as we have found that the cells primoexpanded in CellGro™ Hematopoietic Stem Cell Medium with human serum and supplements had worse first-passage growth than the cells primoexpanded with fetal calf serum, what we considered to be caused by lack of the supplements. The osteogenic media (both the medium with human serum and the medium with fetal calf serum) were changed once a week, while to the medium with human serum, the supplements were added once more in the half of each week. Photographic documentation was performed once a week. After two to four weeks, the scaffolds with the cells were removed from the culture medium and either implanted subcutaneously into immunodeficient mice, or sent for histological, immunohistochemical or electronmicroscopical examination.

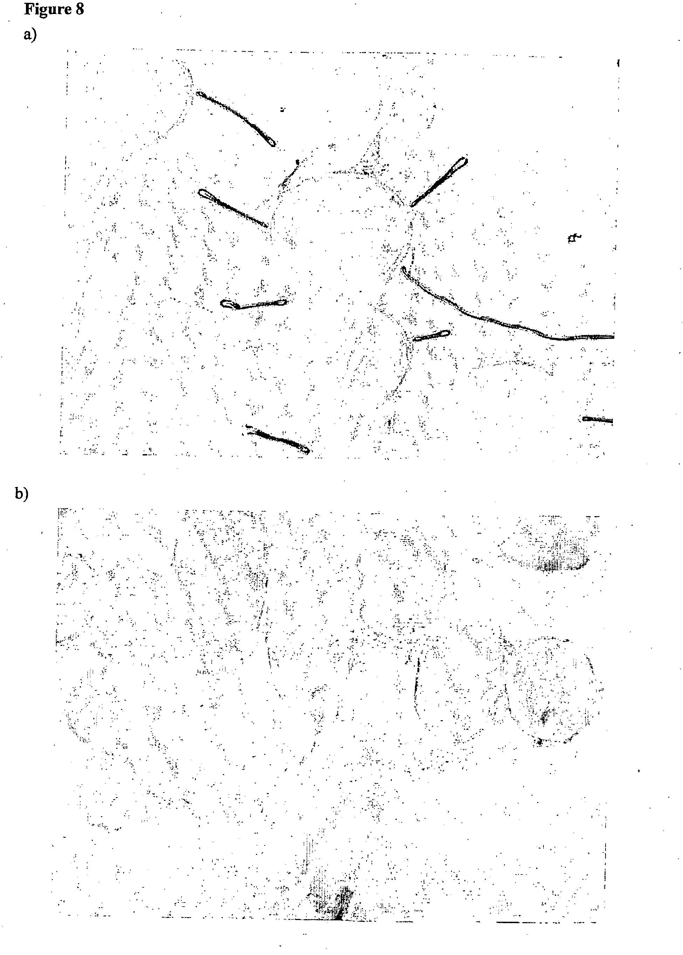

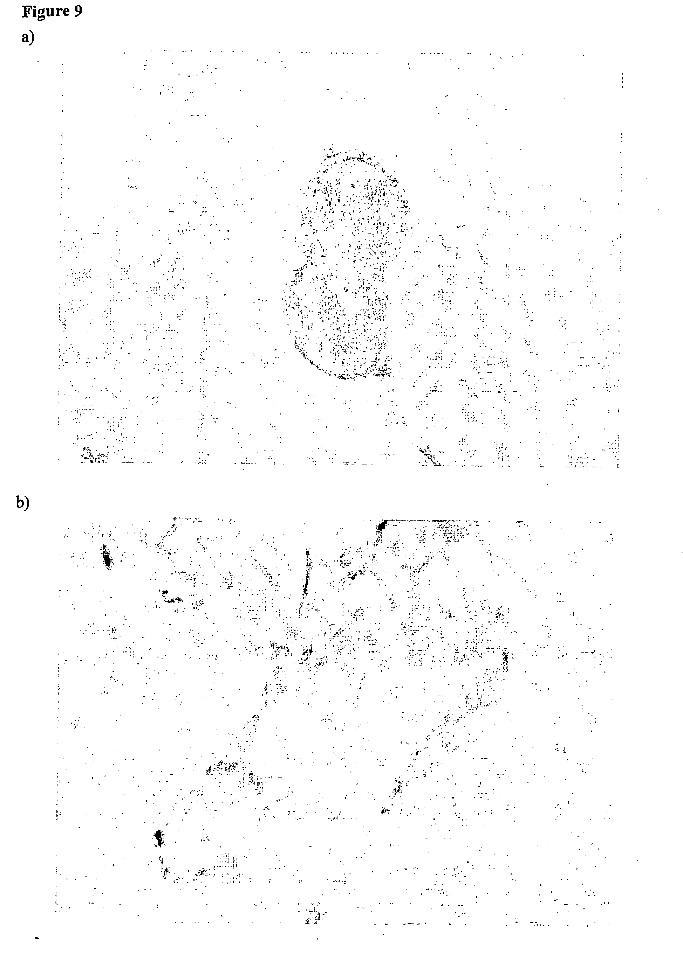

The growth of the cells on the three-dimensional scaffolds is shown in Figure 8. It is clearly visible that, after only few days, the cells on the surface of the scaffold started to bridge the gaps between the fibres (8a) and after two to four weeks they were already present inside the scaffold and produced extracellular matrix (8b). Figures 9 and 10 show the matrix production on histological sections. To prevent the displacement of the cells, the matrix and the polylactide fibres, some of the scaffolds were embedded in synthetic resin and half-thin sections were performed (9a). This procedure leads to numerous artefacts, but the sponge matrix production (small arrow) can be clearly visible besides the cells (large arrow). This procedure, however, prevents certain types of special staining. For these stainings, paraffin-embedded blocks were cut in standard way and glued on a glass slide. This method may lead to a partial loss of polylactide fibres (10b) or displacement of the fibres and the matrix (9b, 10b), but special stainings for osteoid (9b), collagen formation (10a) and osteonectin deposition (10b) may be employed. These proteins or protein mixtures are typical for bone formation but cannot prove whether also mineralization occurs. To get this information, von Kossa staining of the polylactide scaffolds was performed and microphotographs were taken using the stereomicroscope (Fig. 12a). The positivity of von Kossa staining shows calcium deposits not only on the surface of the scaffold, but also in its centre (Figure 12a).

Example 8 - Elemental analysis and bone matrix formation by the cells growing on polylactide carriers

Tissue calcification may be either dystrophic or osteogenic. The dystrophic calcification occurs during calcium deposition in necrotic or fibrous tissues, where calcium forms various organic and inorganic compounds, but not hy-droxyapatite. Hydroxyapatite ($Ca_5(PO_4)_3OH$) is characterized by its crystalline structure and by the ratio of calcium to phosphorus 5:3 when the elemental analysis is performed.

For these reasons, the hydroxyapatite crystals on the three-dimensional carriers were displayed by scanning microscope and the elemental analysis was performed by the same apparatus. The scanning microscope Hitachi at 8.8 keV energy was used. The energy of the repulsed electrons was measured using the Narcom machine by ETMA analysis. In Figure 11a, numerous hydroxyapatite crystals of characteristic appearance are clearly visible. Furthermore, in Figure 11b, the diagram from the ETMA elemental analysis is shown, with characteristic peaks of repulsed electrons at the energy levels of calcium and phosphorus in the ratio of approximately 5:3. These results show that the cells grown in CellGro™ Hematopoietic Stem Cell Medium with 10% human serum and the supplements are able to form all components of bone matrix under osteoinductive conditions and on the three-dimensional scaffolds.

Example 9 - Bone formation from the human cells primo expanded on the three-dimensional polylactide scaffolds in immunodeficient mice

Implantation of the scaffolds with the seeded human cells into the immunodeficient mice was performed as described above. After six to nine weeks after the implantation of the scaffolds with human osteoblasts, the mice were sacrificed and fixed by perfusion fixation with 10% formaldehyde, using blunt needle placed in the left heart chamber with concurrent opening of the right heart chamber. Explants were divided into four parts by two perpendicular cuts across the centre of the scaffold, embedded in formaldehyde, cut into thin slices and stained with Ladewig's modification of Mason's trichome. This staining provides for the differentiation of the mineralized bone matrix (red) from the osteoid (nonmineralized matrix - blue) and eventual dystrophic tissues (amyloid etc.), which stain pink. The representative result is shown in Figure 12. This figure shows that the mineralization of the explant did not start on the surface, but in the centre of the scaffold and the lamellar bone apposition is visible. On the surface of the scaffold, blue nonossified osteoid is still visible. No inflammatory or giant cell reaction is present, which means that the polylactide scaffolds were well tolerated.

Example 10 - Cultivation in a bioreactor

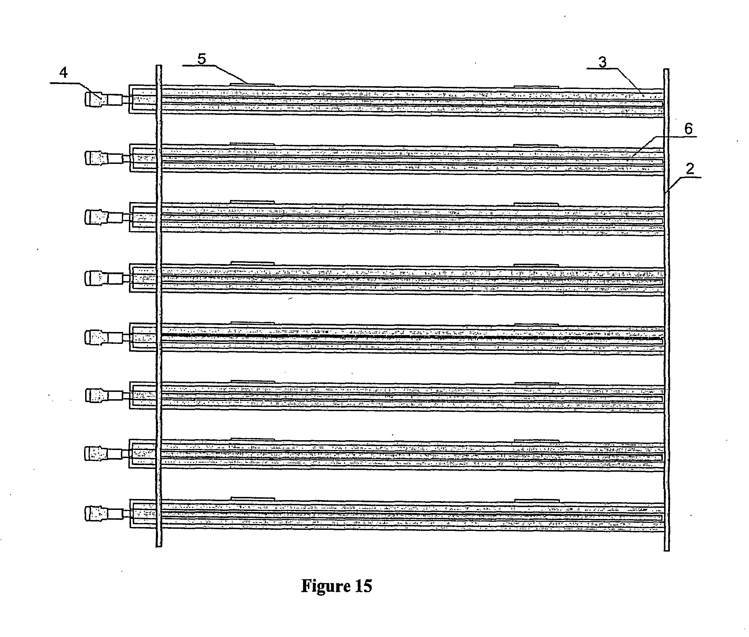

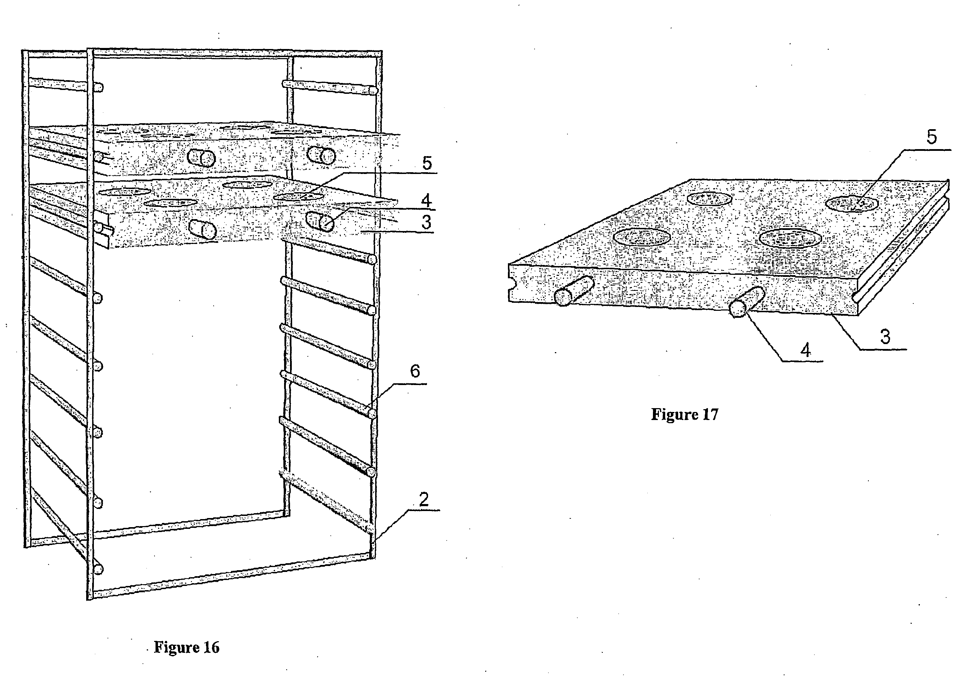

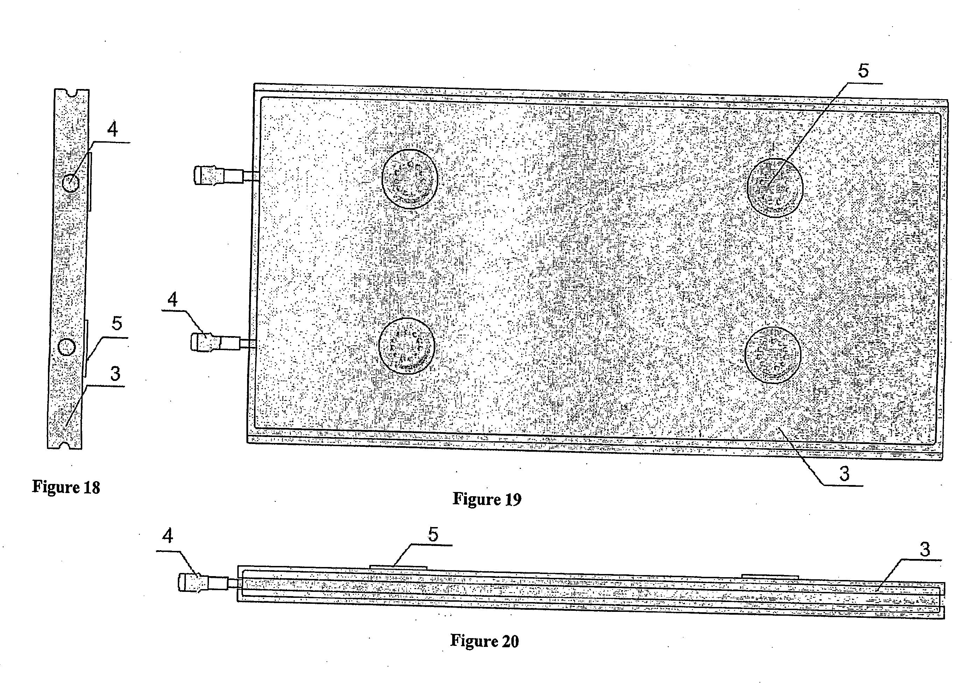

The basis of the bioreactor is a cassette system 1, consisting of a carrier 2 and cast plastic cultivation vessels 3, having inlets 4, placed at the front side, on which silicon rubber septa, similar to that on plastic transfusion bags or bags for the cultivation of cells *in vitro* in suspension, are fused (Fig. 17-20). The cultivation vessels 3 are of rectangular shape with the dimensions of 30x15 cm and 1.5-2 cm height. These dimensions follow the dimensions of standard inverted microscope tables for the cultivation of cells. In the upper part of the cultivation vessel, filters 5 from a material providing for sterile gas exchange between the inner atmosphere of the vessel and the atmosphere of the $CO_2$ incubator, and at the same time preventing the intrusion of infectious germs (filters having the diameter of pores 0.22 $\mu$m are commonly used). In the side parts of the cultivation vessel, grooves 7 are pressed (Fig. 20), facilitating its insertion into the metal carrier 2 (Fig. 16), consisting of two rectangular frames 8 connected with supporting wires 6, placed perpendicularly to the frames 8 and attached to the side parts of the frames in even distances. The frames 8 and the supporting wires 6 are made of welded wires of appropriate constitution and diameter. Wires from stainless steel or heat-treated or alloy-treated steel or copper wires or wires from any other suitable and practical material can be used. The copper wires gain an antibacterial activity after undergoing slight corrosion. The overall perspective view of the cassette system 1 is shown in Fig. 13, the front view in Fig. 14 and the side view in Fig. 15.

Seeding of the cells into the cultivation vessel 3 is carried out by the following procedure: an appropriate amount of the cells is applied with a sterile syringe through one inlet 4 with consequent aspiration of the gas with another syringe through the second inlet 4 to release the overpressure. The same process is used for adding the complete medium into the cultivation vessel 3. It is desirable that the bottom of the cultivation vessel 3 is covered with at least two, but preferably three mm of the solution, that for the surface of the bottom of the cultivation vessel equal to 150 $cm^2$ requires approx. 30-45 ml of the complete medium for one cultivation vessel. 50 ml sterile syringes certified for the clinical use can therefore be used. For preserving the sterility, it is desirable that the complete medium is distributed in flasks of these volumes or their multiples and the flasks should have a shape allowing for aspiration of the solution with a syringe and a needle, like standard infusion flasks. Subsequently, the supplements are added to the cultivation vessel. Similarly as for infusion solutions it is desirable (although not necessary) that the supplements are supplied in aliquots, either mixed in appropriate ratios or pure, either in solution or lyophilized.

After seeding the cells and the complete medium and adding the supplements, the cultivation vessels are placed into the carrier 2 and the cassette system 1 is inserted into $CO_2$ incubator. The supplements are added twice a week. With regard to the construction of the bioreactor and the closed cultivation system, the cultivation vessel can be withdrawn under appropriate sterile conditions and examined by the inverted microscope whether sufficient growth of adherent cell layer has occurred. After achieving the optimal amount of cells (the proposed cultivation period is 2-3 weeks, in our laboratory we have obtained the optimal amount of cells after the cultivation period of 13-17 days), the cells can be harvested.

During the harvest, the complete medium with non-adherent cells is removed by a sterile syringe and injection needle in the first step, while a second syringe pumps in the air to prevent the underpressure. Aliquots of the complete medium are then sent to microbiologic cultivation and to pyrogens and endotoxin determination. These aliquots can be sampled also in the course of the cultivation, if it is necessary or desirable, because the volumes of the aliquots are not large. The adherent cells are then incubated with a small amount of 1% EDTA and 0.06% trypsin solution to be detached from the surface of the plastic vessel. Trypsin is the only animal peptide that is contacted with the cells during the whole cultivation procedure. The 1% EDTA and 0.05% trypsin solution (Invitrogen) contains porcine trypsin in very low concentration. After the neutralization with the complete medium with human serum and further washing with the same medium, the possibility of allergic reaction to the animal protein is extremely low. The virological safety of the product can be increased by 25 Gy irradiation or an alternative enzyme, similar to trypsin, which does not require neutralization, is not an animal peptide and is virologically safe, can be used (such as TrypLE™ Select or TrypLE™ Express, Invitrogen Inc.).

After dispatching the cells from the surface of the plastic cultivation vessel, a larger amount of the complete medium with human serum is added, which leads to the decrease of the loss of the cells during aspiration and eventually to the neutralization of trypsin. The adherent cells are then removed with sterile syringe and injection needle, with consequent pumping in the air through the second inlet, so that stable pressure is retained. In this case, it is desirable to use as thin needle as possible for the aspiration of the cell suspension, so that the cell clusters dissociate and a unicellular suspension is formed. The adherent cells are then inserted into children's transfusion bag, centrifuged, washed with the complete medium and prepared for the application. All manipulations with the cells occur in the closed system and in the appropriate biohazard box.

The method of the invention can be useful in the preparation of mesenchymal cells also for other than orthopaedic use. In such a case, when the presence of the CD45+ cells is undesirable, they can be removed with the aid of the immunomagnetic antibody against CD45+ (e.g. in CliniMACS™, Miltényi Biotec, which is certified for the clinical use and works also as a closed system).

**Claims**

1. A method of in vitro cultivation of human mesenchymal stem cells, particularly for the treatment of non-healing fractures, from mononuclear marrow blood cells, **characterized in that** the cultivation of the cells is carried out in a single step in closed system, in a medium certified for the clinical use, with an addition of 10% human serum and supplements, which are dexamethasone, ascorbic acid, human recombinant insulin, human recombinant PDGF-BB, human recombinant EGF in combination with human recombinant M-CSF and human recombinant FGF-2, without animal proteins, without removal of hematopoietic cells and without medium exchange during the cultivation procedure under the standard conditions for the cultivation of tissue cultures.

2. The method of in vitro cultivation of human mesenchymal stem cells according to claim 1, **characterized in that** CellGro™ Hematopoietic Stem Cell Medium is used as the medium certified for the clinical use.

3. The method of in vitro cultivation of human mesenchymal stem cells according to any of claims 1 or 2, **characterized in that** the supplements are added at least once during the cultivation procedure.

4. The method of in vitro cultivation of human mesenchymal stem cells according to any of claims 1 to 3, **characterized in that** the cultivation of the cells is performed for one to three weeks.

5. The method of in vitro cultivation of human mesenchymal stem cells according to claim 4, **characterized in that** the cultivation of the cells is performed for 13 to 17 days.


**Patentansprüche**

1. Verfahren zur in-vitro Kultivierung von menschlichen mesenchymalen Stammzellen, insbesondere zur Behandlung nichtheilender Brüche, aus mononukleären Zellen des Markblutes, **dadurch gekennzeichnet, dass** die Zellkultivierung in einem, für die klinische Verwendung genehmigten Medium, in einem geschlossenen System in einer Stufe mit dem Zusatz von dem 10 %-igen Humanserum und Supplements, wie Dexamethason, Ascorbinsäure, rekombinantes Human-Insulin, rekombinantes humanes PDGF-BB, rekombinantes humanes EGF in Kombination mit dem rekombinanten humanen M-CSF und dem rekombinanten humanen FGF-2, ohne tierische Eiweiße, ohne die Beseitigung von hämopoetischen Zellen und ohne Mediumwechsel während der Kultivierung unter den üblichen Bedingungen für die Kultivierung von Gewebekulturen, durchgeführt wird.

2. Verfahren zur in-vitro Kultivierung von menschlichen mesenchymalen Stammzellen nach dem Anspruch 1, **dadurch gekennzeichnet, dass** als das für die klinische Verwendung genehmigte Medium das CellGro™ hematopoietic stern cell medium verwendet wird.

3. Verfahren zur in-vitro Kultivierung von menschlichen mesenchymalen Stammzellen nach einem der Ansprüche 1 oder 2, **dadurch gekennzeichnet, dass** während der Zellkultivierung mindestens einmal Supplements hinzugefügt werden.

4. Verfahren zur in-vitro Kultivierung von menschlichen mesenchymalen Stammzellen nach einem der Ansprüche 1 bis 3, **dadurch gekennzeichnet, dass** die Zellkultivierung in der Dauer von einer bis drei Wochen vorgenommen wird.

5. Verfahren zur in-vitro Kultivierung von menschlichen mesenchymalen Stammzellen nach dem Anspruch 4, **dadurch gekennzeichnet, dass** die Zellkultivierung in der Dauer von 13 bis 17 Tagen durchgeführt wird.


**Revendications**

1. Procédé de culture in vitro des cellules souches mésenchymateuses humaines, notamment pour le traitement des fractures à cicatrisation difficile, à partir des cellules mononucléaires du sang de la moelle, **caractérisé en ce que** la culture des cellules se fait en une seule étape dans un système clos, dans un milieu certifié pour utilisation clinique, avec l'addition de 10% de sérum humain et de suppléments, à savoir la dexaméthasone, l'acide ascorbique, l'insuline humain recombinant, le PDGF-BB humain recombinant, l'EGF humain recombinant en combinaison avec le M-CSF humain recombinant et le FGF-2 humain recombinant, sans protéines animales, sans l'élimination des cellules hémopoïétiques et sans l'échange du milieu durant la culture sous les conditions standards pour la culture des cultures tissulaires.

**2.** Procédé de culture in vitro des cellules souches mésenchymateuses humaines selon la revendication 1, **caractérisé en ce que** CellGro™ Hematopoietic Stem Cell Medium est utilisé comme le milieu certifié pour l'utilisation clinique.

**3.** Procédé de culture in vitro des cellules souches mésenchymateuses humaines selon l'une quelconque des revendications 1 ou 2, **caractérisé en ce que** les suppléments sont ajoutés au moins une fois durant la procédure de culture des cellules.

**4.** Procédé de culture in vitro des cellules souches mésenchymateuses humaines selon l'une quelconque des revendications 1 à 3, **caractérisé en ce que** la culture des cellules est effectuée pendant une période d'une à trois semaines.

**5.** Procédé de culture in vitro des cellules souches mésenchymateuses humaines selon la revendication 4, **caractérisé en ce que** la culture des cellules est effectuée pendant une période de 13 à 17 jours.

**Figure 1**

a)

b)

**Figure 2**

a)

b)

## Figure 3

R1 = CD45$^+$ = 28,8%

R2 = CD235a$^+$ = 1,0%

R3 = CD45$^-$ CD235a$^-$ = 70,2%

R4 (gate: R3): 65,3% of CD45$^-$ CD235a$^-$ cells are CD90$^+$

**Figure 4**

□1 alpha-MEM + 10% fetal calf serum

□2 alpha-MEM + 10% human serum

□3 alpha-MEM + 10% human serum + 5 supplements (Gronthos-Simmons

□4 alpha-MEM + 10% human serum + 5 supplements + M-CSF

□5 alpha-MEM + 10% human serum + 5 supplements + M-CSF + FGF-2

■6 CellGro$^{TM}$ + 10% human serum + 7 supplements

□7 CellGro$^{TM}$ + 10% autologous plasma + 7 supplements

Figure 5

*) p <0,05

■  Cells primoexpanded in CellGro<sup>TM</sup> with human serum and 7 supplements

☐  Cells primoexpanded in CellGro<sup>TM</sup> with human serum and 7 supplements
    followed by CD45 cell depletion

☐  Cells primoexpanded in alpha-MEM with fetal calf serum

**Figure 6**

a)

b)

**Figure 7**

a)

b)

**Figure 8**

a)

b)

**Figure 9**

a)

b)

**Figure 10**

a)

b)

**Figure 11**

a)

S4700 10.0kV 12.7mm ×11.0k SE(M)    5.00um

b)

**Figure 12**

a)

b)

Figure 14

Figure 13

Figure 15

38

Figure 17

Figure 16

Figure 19

Figure 20

Figure 18

**EP 1 999 250 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Csongradi JJ ; Maloney WJ.** Ununited lower limb fractures. *West J Med,* 1989, vol. 150, 675-680
- **Chen F et al.** Smoking and bony union after ulna-shortening osteotomy. *Am. J. Orthop.,* 2001, vol. 30, 486-489
- **Foulk DA ; Szabo RM.** Diaphyseal humerus fractures: natural history and occurrence of nonunion. *Orthopedics,* 1995, vol. 18, 333-335
- **Nilsson LT et al.** Factors predicting healing complications in femoral neck fractures. 138 patients followed for 2 years. *Acta Orthop. Scand.,* 1993, vol. 64, 175-177
- **Heetveld MJ et al.** Internal fixation for displaced fractures of the femoral neck. Does bone density affect clinical outcome?. *J Bone Joint Surg Br,* 2005, vol. 87, 367-373
- **Sarmiento A et al.** Factors influencing the outcome of closed tibial fractures treated with functional bracing. *Clin Orthop.,* 1995, vol. 315, 8-24
- **Bhandari M, et al.** A lack of consensus in the asessment of fracture healing among orthopaedic surgeons. *J. Orthop. Trauma.,* 2002, vol. 16, 562-566
- Tibial shaft fractures. **Bucholz RW.** Orthopaedic Decision Making. 1996, 74
- Tibial shaft fractures. **Gorczyca JT.** Review of Orthopaedic Trauma. Saunders, 2001, 127
- **Green SA et al.** External fixation for the uninfected angulated nonunion of the tibia. *Clin Orthop.,* 1984, vol. 190, 204-211
- **Wang JW ; Weng LH.** Treatment of distal femoral nonunion with internal fixation, cortical allograft struts, and autogenous bone-grafting. *J Bone Joint Surg Am.,* 2003, vol. 85, 436-440
- **Ilizarov GA et al.** Treatment of closed diaphysial fractures of long tubular bones by means of transosseous osteosynthesis. *Sov Med.,* 1983, vol. 9, 21-24
- **Connoly JF et al.** Autologous marrow injection for delayed unions of the tibia: a preliminary report. *J Orthop Trauma.,* 1989, vol. 3, 276-282
- **Hernigou P et al.** Percutaneous autologous bone-marrow grafting for nonunions. *J. Bone Joint Surg Am.,* 2005, vol. 87, 1430-1437
- **Werntz JR et al.** Qualitative and quantitative analysis of orthotopic bone regeneration by marrow. *J Orthop Res.,* 1996, vol. 14, 85-93

- **Bruder SP et al.** The effect of implants loaded with autologous mesenchymal stem cells on the healint of canine segmental bone defects. *J Bone Joint Surg Am.,* July 1998, vol. 80, 985-996
- **Jiang Y.** Pluripotency of mesenchymal stem cells derived from adult marrow. *Nature,* 2002, vol. 418, 41-49
- **Pittenger MF et al.** Multilineage potential of adult human mesenchymal stem cells. *Science,* 1999, vol. 284, 143-147
- **Reyes M et al.** Purification and ex vivo expansio of postnatal human marrow mesodermal progenitor cells. *Blood,* 2001, vol. 98, 2615-2625
- **Makino S et al.** Cardiomyocytes can be generated from marrow stromal cells in vitro. *J Clin Invest.,* 1999, vol. 103, 697-705
- **Pittenger MF ; Martin BJ.** Mesenchymal stem cells and their potential as cardiac therapeutics. *Circ Res.,* 2004, vol. 95, 9-20
- **Jendelová P ; Herynek V ; DeCroos J et al.** Imaging the fate of implanted bone marrow stromal cells labeled with superparamagnetic nanoparticles. *Magn Reson Med,* 2003, vol. 50, 767-776
- **DiGirolamo CM et al.** Propagation and senescence of human marrow stromal cells in culture: a simple colony-forming assay identifies samples with the greatest potential to propagate and differentiate. *Br JHaematol.,* 1999, vol. 107, 275-281
- **Colter DC et al.** Rapid expansion of recycling stem cells in cultures of plastic-adherent cells from human bone marrow. *Proc Natl Acad Sci USA.,* 2000, vol. 97, 3213-3218
- **Coelho MJ ; Trigo Cabral A ; Fernandes MH.** Human bone cell cultures in biocompatibility testing. Part I: osteoblastic differentiation of serially passaged human bone marrow cells cultured in α-MEM and in DMEM. *Biomaterials,* 2000, vol. 21, 1087-1094
- **Mackensen A et al.** Presence of IgE antibodies to bovine serum albumin in a patient developing anaphylaxis after vaccination with human peptide-pulsed dendritic cells. *Cancer Immunol Immunother.,* 2000, vol. 49, 152-156
- **Sugiura F ; Kitoh H ; Ischiguro N.** Osteogenic potential of rat mesenchymal stem cells after several passages. *Bioch Bioph Res Comm.,* 2004, vol. 316, 233-239

41

- **Gronthos S ; Simmons PJ.** The growth factor requirements fo STRO-1-positive human bone marrow stromal precursors under serum-deprived conditions in vitro. *Blood,* 1995, vol. 85, 929-940
- **Jin-Xiang F et al.** Homing efficiency and hematopoietic reconstitution of bone marrow-derived stroma cells expanded by recombinant human macrophage-colony stimulating factor in vitro. *Exp Hematol,* 2004, vol. 32, 1204-1211
- **Tsutsumi S et al.** Retention of multilineage differentiation potential of mesenchymal cells during proliferation in response to FGF. *Bioch Bioph Res Comm,* 2001, vol. 288, 413-419
- **Hankey DP et al.** Enhacement of human osteoblast proliferation and phenotypic expression when cultured in human serum. *Acta Orthop Scand.,* 2001, vol. 72, 395-403
- **Stute N et al.** Autologous serum for isolation and expansion of human mesenchymal stem cells for clinical use. *Exp Hematol.,* 2004, vol. 32, 1212-1225
- **Schecroun N ; Delloye Ch.** In vitro growth and osteoblastic differentiation of human bone marrow stromal cells supported by autologous plasma. *Bone,* 2004, vol. 35, 517-524
- **Kim SJ et al.** *Biochemical and Biophysical Research Communications,* 2005, vol. 329, 25-31
- **Kuznetsov SA ; Mankani MH ; Robey PG.** Effect of serum on human bone marrow stromal cells: ex vivo expansion and in vivo bone formation. *Transplantation,* 2000, vol. 70, 1780-1787
- **Baksch D ; Davies JE ; Zandstra PW.** Adult human bone marrow-derived mesenchymal progenitor cells are capable of adhesion-independent survival and expansion. *Exp Hematol.,* 2003, vol. 31, 723-732
- **Baksch D ; Davies JE ; Zandstra PW.** Soluble factor cross-talk between human bone marrow-derived hematopoietic and mesenchymal cells enhances in vitro CFU-F and CFU-O growth and reveals heterogeneity in the mesenchymal progenitor cell compartment. *Blood,* 2005, vol. 106, 3012-3019
- **Taichman RS.** Blood and bone: two tissues whose fates are intertwined to create the hematopoietic stem-cell niche. *Blood,* 2005, vol. 105, 2631-2639
- **Soenen-Cornu V et al.** Mesenchymal cells generated from patients with myelodysplastic syndromes are devoid of chromosomal clonal markers and support short- and long-term hematopoiesis in vitro. *Oncogene,* 2005, vol. 24, 2441-2448
- **Hernigou P et al.** Percutaneous autologous bone-marrow grafting for nonunions. *J Bone Joint Surg Am.,* 2005, vol. 87, 1430-1437